# FASCICULE DE BREVET EUROPEEN

(11) **EP 1 556 489 B1**
(45) Date de publication et mention de la délivrance du brevet: **26.01.2011**
(21) Numéro de dépôt: 03767876.0
(22) Date de dépôt: 31.10.2003
(51) Int. Cl.: C12N 15/12, C07K 14/705, G01N 33/50, A61K 38/18

(54) **COMPOSITION PHARMACEUTIQUE POUR LA PREPARATION D'UN MEDICAMENT DESTINE A TRAITER ET/OU A PREVENIR UNE PATHOLOGIE LIEE A UNE CONDUITE OBSESSIONNELLE OU L'OBESITE**
PHARMAZEUTISCHE ZUSAMMENSETZUNG ZUR HERSTELLUNG EINES MEDIKAMENTS ZUR BEHANDLUNG UND/ODER VORBEUGUNG EINER PATHOLOGIE DIE MIT EINER OBSESSIVEN STÖRUNG UND/ODER FETTLEIBIGKEIT IM ZUSAMMENHANG STEHT
PHARMACEUTICAL COMPOSITION FOR PREPARING A MEDICINE FOR TREATING AND/OR PREVENTING A PATHOLOGY RELATED TO AN OBSESSIVE BEHAVIOUR OR OBESITY

(30) Priorité: 31.10.2002 FR 0213725
(43) Date de publication de la demande: 27.07.2005
(73) Titulaire: CENTRE NATIONAL DE LA RECHERCHE SCIENTIFIQUE (CNRS), 75794 Paris Cedex 16 (FR)
(72) Inventeur: COMPAN, Valérie, Résidence les Jeux d'O, 34094 Montpellier cedex 05 (FR)
(74) Mandataire: Novagraaf Technologies
(86) Numéro de dépôt international: PCT/FR2003/003262
(87) Numéro de publication internationale: WO 2004/042063

(56) Documents cités:
- WO-A-00/64441
- WO-A-00/77199
- WO-A-02/06272
- WO-A-94/09828
- WO-A-94/14957
- BOCKAERT J: "New view on the ligand-receptor interaction." EUROPEAN NEUROPSYCHOPHARMACOLOGY, vol. 13, no. Supplement 4, septembre 2003 (2003-09), page S159, XP009027353 16th Congress of the European College of Neuropsychopharmacology;Prague, Czech Republic; September 20-24, 2003 ISSN: 0924-977X (ISSN print)
- MCMAHON, LANCE R. ET AL: "Antagonism of 5-hydroxytryptamine4 receptors attenuates hyperactivity induced by cocaine: putative role for 5-hydroxytryptamine4 receptors in the nucleus accumbens shell" JOURNAL OF PHARMACOLOGY AND EXPERIMENTAL THERAPEUTICS (1999), 291(1), 300-307, 1999, XP002329176
- WADA, YUJI ET AL: "Action of the selective 5- HT4 receptor antagonist SB 204070A in the rat kindling model of epilepsy" NEUROSCIENCE RESEARCH COMMUNICATIONS (1999), 25(1), 43-49, 1999, XP009027451
- VAN DEN WYNGAERT ILSE ET AL: "Cloning and expression of a human serotonin 5-HT-4 receptor cDNA." JOURNAL OF NEUROCHEMISTRY, vol. 69, no. 5, 1997, pages 1810-1819, XP002247071 ISSN: 0022-3042
- BENDER E ET AL: "STRUCTURE OF THE HUMAN SEROTONIN 5-HT4 RECEPTOR GENE AND CLONING OF A NOVEL 5-HT4 SPLICE VARIANT" JOURNAL OF NEUROCHEMISTRY, NEW YORK, NY, US, vol. 74, no. 2, février 2000 (2000-02), pages 478-489, XP000953119 ISSN: 0022-3042

## Description

La présente invention concerne le domaine du traitement et de la prévention des maladies liées à des conduites obsessionnelles telles que l'anorexie, la boulimie et l'addiction aux drogues d'abus, associées ou non au stress ainsi que le domaine du traitement et de la prévention de l'obésité. La présente invention concerne plus particulièrement l'utilisation d'un ligand du récepteur 5-HT₄ pour la préparation d'un médicament destiné à traiter et/ou prévenir lesdites maladies tel que la boulimie, l'anorexie, l'addiction aux drogues d'abus et/ou l'obésité.

Les troubles alimentaires coexistent avec l'anxiété et la dépression et représentent une inquiétude croissante des pays développés (Garrow, 1991 ; Kuczmarski et al., 1994). Comme pour la plupart des pathologies neuropsychiatriques, la combinaison de l'influence des facteurs de l'environnement et d'une prédisposition génétique paraît responsable de ces déficits comportementaux (Fairburn et al., 1998 ; Lilenfeld et al., 1998 ; Barsh et al., 2000). Les mères de 75 % des femmes anorexiques souffrent de dépression ou sont alcooliques, un an avant l'expression des symptômes de leur enfant. Le syndrome anorexique est aussi plus fréquemment détecté dans une même famille que dans la population générale sans qu'aucun gène lié à cette pathologie n'ait été identifié. Les conduites boulimiques, souvent concomitantes de l'anorexie chez un même individu, sont caractérisées par des phases impulsives et répétées d'ingestion d'une quantité élevée d'aliments.

La boulimie est ainsi classée parmi les conduites liées à l'addiction (Définitions Internationales de Psychiatrie, DSM). L'aliment peut être considéré comme une récompense dont l'obtention repose sur la volonté (« wanting » : appetite/incentive motivation ») et est motivée par une composante liée à l'hédonisme (« Liking : pleasure/palatability ») (Hoebel, 1997 ; Salamone et al., 1997 ; Stratford and Kelley, 1997 ; Stratford and Kelley, 1999). L'excès ou l'absence de prise d'aliments n'est pas seulement limité aux déficits métaboliques et/ou endocriniens, mais dépend aussi du stress (Donohoe, 1984 ; Morley et al., 1983 ; Vergoni and Bertolini, 2000), de l'anxiété (Godart et al., 2000) et de la dépression (Viesselman and Roig, 1985 ; Casper, 1998).

L'hypothalamus, l'amygdale et l'hippocampe sont impliqués dans la régulation de la consommation d'aliments. En outre, l'activité des neurones du noyau accumbens est modifiée lors de l'anticipation ou après l'obtention d'une récompense classique comme l'aliment ou les drogues d'abus (Di Chiara, 1995 ; Hoebel, 1997 ; Koob and Nestler, 1997 ; Salamone et al., 1997).

Bien qu'une émergence de découvertes montre les implications de nombreux peptides dans la régulation des comportements alimentaires (Leptine, Orexins/hypocretins, CART, NPY, POMC, CRH, TRH), les influences des neuromédiateurs classiques comme la sérotonine (5-HT) et la dopamine (DA) restent incontournables. Le GABA et le glutamate sont aussi à considérer (Taber and Fibiger, 1997 ; Kelley and Swanson, 1997 ; Stratford and Kelley, 1997 ; Stratford et al., 1998).

Les systèmes dopaminergiques du noyau accumbens sont impliqués dans l'anticipation d'une récompense (drogues d'abus, aliments). Une administration chronique de clozapine, antagoniste des récepteurs de la DA, induit une hyperphagie (Ackerman and Nolan, 1998 ; Allison et al., 1999). La cocaïne et l'amphétamine, connues pour augmenter la transmission de la DA, sont anorexigènes (Foltin and Evans, 1999).

Cependant, les systèmes sérotoninergiques restent un maillon inévitable qui contrôle la prise alimentaire (Barnes and Sharp, 1999) du fait de l'utilisation de la fenfluramine, inhibiteur de la capture de la sérotonine chez des patients obèses (Guy - Grand B, 1995).

En résumé, des déficits des combinaisons d'interactions entre des facteurs de l'environnement (stress) et des facteurs génétiques (gènes codant des récepteurs présents dans le cerveau) apparaissent responsables des troubles comportementaux comme la boulimie, l'anorexie ou l'addiction aux drogues d'abus. Ces pathologies, et de manière plus évidente, la boulimie est aujourd'hui pensée comme une conduite toxicomane.

Au plan neurobiologique, l'état de nos connaissances favorise l'intervention combinée de plusieurs systèmes neuronaux pour réguler les comportements alimentaires. Les plus connus sont les systèmes sérotoninergiques qui expriment le messager cérébral (neuromédiateur) qu'est la 5-HT. Les aires cérébrales où leurs actions se manifestent sont principalement l'hypothalamus, l'amygdale et le noyau accumbens.

La relation exacte entre les effets du stress et la 5-HT est rendue complexe par les influences réciproques entre les activités des systèmes sérotoninergiques et de l'axe hypothalamo-hypophysaire (Chaouloff F., 2000). En revanche, l'application d'un stress entraîne des augmentations de la transmission sérotoninergique.

Le stress provoque des élévations de la transmission sérotoninergique. Les paradigmes expérimentaux où le stress est associé à une peur conditionnée entraînent une augmentation du métabolisme et de la libération de la 5-HT dans le cortex préfrontal médian (Adell et al., 1997 ; Inoue et al., 1994), le noyau accumbens (Inoue et al., 1994 ; Ge et al., 1997), l'amygdale (Amat et al., 1998) et l'hippocampe dorsal (Ge et al., 1997 ; Joseph and Kennett, 1983). En particulier, le stress de contrainte (immobilisation forcée) augmente le renouvellement de la 5-HT dans l'hypothalamus et l'amygdale du rat et de la souris (Konstandi et al., 2000). De la même manière, l'action du « Corticotropin Releasing hormone or Factor » (CRF) sur les neurones sérotoninergiques du système corticomésolimbique pourrait modifier les taux de la 5-HT (Lowry et al., 2000 ; Price and Lucki, 2001). En outre, des altérations du fonctionnement des récepteurs des glucocorticoïdes entraînent des variations de la concentration de la 5-HT dans le noyau accumbens (Sillaber et al., 1998). De plus, l'injection répétée de corticostérone augmente l'activation des neurones de l'hippocampe (CA1) induite par un agoniste du récepteur 5-HT₄ (Zahorodna et al., 2000). Finalement, de nombreuses études supposent que le CRF est responsable de l'effet anorexigène d'un stress. En particulier, l'injection intracérébroventriculaire de CRF induit une diminution de la prise alimentaire chez la souris (Momose et al., 1999).

La sérotonine inhibe la prise alimentaire. Les approches pharmacologiques combinées aux stratégies de la transgenèse indiquent que les récepteurs 5-HT_{1A/1B} et 5-HT_{2A/2C} sont impliqués dans la régulation de la prise alimentaire et, par ailleurs, du stress (Bonasera et Tecott, 2000 ; Bouwknecht et al., 2001 ; Dourish et al., 1986 ; Heisler et al., 1998 ; Lucas et al., 1998 ; Samanin et Garattini, 1996). L'anorexie liée au stress est alors supposée résulter de l'augmentation de l'activité des neurones sérotoninergiques. De nombreuses études attribuent l'effet anorexigène de la fenfluramine à l'activation du récepteur 5-HT_{1B} alors que celle du récepteur 5-HT_{1A} (autorécepteur), inhibitrice de la libération de 5-HT, induit une élévation de la prise alimentaire. L'insensibilité des souris dépourvues du récepteur 5-HT_{1B} à l'injection de la fenfluramine confirme son implication dans la régulation de la prise alimentaire (Lucas et al., 1998). Les récepteurs 5-HT_{2C} interviennent aussi dans la consommation d'aliments car les souris qui en sont privées s'avèrent obèses (Heisler et al., 1998). La leptine est connue pour diminuer la prise alimentaire, mais elle n'est pas associée à cette obésité (Nonogaki et al., 1998).

Une étude récente montre que les récepteurs 5-HT_{2C} sont aussi responsables de l'effet anorexigène de la fenfluramine (Vickers et al., 2001). Enfin, l'administration de tropisétron, antagoniste des récepteurs 5-HT₃ et 5-HT₄, augmente la prise alimentaire d'un régime modifié par un seul acide aminé (Erecius et al., 1996) mais cet effet a été attribué au récepteur 5-HT₃ (Jiang and Gietzen, 1994). Par conséquent, aucune donnée n'est aujourd'hui disponible sur la contribution du récepteur 5-HT₄ dans la prise alimentaire.

En résumé, l'hypothèse courante pour expliquer qu'un stress diminue la prise alimentaire repose sur deux séries d'études parallèles. La première décrit que le stress augmente l'activité de l'axe hypothalamo-hypophysaire ("stress axis") et des neurones sérotoninergiques. En outre, l'hyperactivité de l'axe hypothalamo-hypophysaire provoque l'augmentation des taux d'hormones comme le CRF, l'urocortin et, en une ultime étape, de la corticostérone. La deuxième série d'analyses montre que les hormones de l'axe du stress et la 5-HT inhibent la prise alimentaire.

En conséquence, de nombreux auteurs proposent la séquence d'évènements suivante :

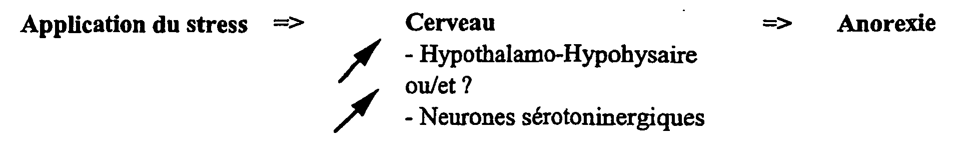

L'ensemble des récepteurs de la 5-HT sont couplés aux protéines G à l'exception du récepteur 5-HT₃ qui est un canal ionique (Saudou and Hen, 1994). Les récepteurs 5-HT_{1A}, 5-HT_{1B}, 5-HT_{1D}, 5-HT_{1E}, et 5-HT_{1F} sont couplés négativement à l'adénylate cyclase et présentent une forte affinité pour la 5-HT. L'activation des récepteurs 5-HT₂ stimule l'activité de la phospholipase C (5-HT_{2A/2C}). Les autres récepteurs de la 5-HT sont positivement couplés à l'adénylate cyclase et incluent le 5-HT₄, 5-HT_{drol} chez la drosophile, et les récepteurs 5-HT₆ et 5-HT₇ chez les mammifères. Le récepteur 5-HT₄ a été décrit pour la première fois dans les colliculi (Dumuis et al., 1988) et sa stimulation provoque une élévation des taux d'AMPc dans l'hippocampe, le cortex cérébral, l'atrium et l'oesophage. Chez l'homme, neuf sous-types de récepteurs 5-HT₄ nommés 5-HT_{4A}, 5-HT_{4B}, 5-HT_{4C}, 5-HT_{4D}, 5-HT_{4E}, 5-HT_{4F}, 5-HT_{4G}, 5-HT_{4BH} et 5-HT_{4N} diffèrent par leur extrémité C-terminale (Bockaert et al., 2003, sous presse, pour revue). Le système de transduction des récepteurs 5-HT_{5A} est associé positivement à l'adénylate cyclase. Celui du 5-HT_{5B} n'a pas encore été identifié.

Les influences fonctionnelles des récepteurs 5-HT₄ ont été très étudiées dans le tractus gastrointestinal, mais peu de données sont disponibles sur leurs contributions dans le cerveau. De l'ensemble des structures de l'encéphale des rongeurs et de l'homme, les plus fortes densités du récepteur 5-HT₄ sont détectées dans le système limbique (Waeber et al., 1994). En particulier, sa concentration est trois fois plus élevée dans le « shell » que dans le « core » du noyau accumbens (Compan et al., 1996). Dans le cerveau des rongeurs, leur taux varie durant le développement et n'atteint leur niveau adulte qu_{'}au 21^{ième} jour après la naissance (Waeber et al., 1994). De l'encéphale de rat, les taux des ARNm codant le récepteur 5-HT₄ sont les plus élevés dans le système olfactif, le striatum, le noyau accumbens, l'habénula et l'hippocampe (Gerald et al., 1995; Ulmer et al., 1996; Vilaro et al., 1996).

Des agonistes des récepteurs 5-HT₄ provoquent une diminution des déficits de mémorisation et améliore l'apprentissage par la mise en jeu de la transmission de l'acétylcholine (Bockaert J et al., 1998). Il est tentant de supposer que le récepteur 5-HT₄ puisse prendre part aux mécanismes neuronaux du noyau accumbens liés à l'apprentissage alimentaire. Quatre études pharmacologiques ont fait état d'une faible contribution du récepteur 5-RT₄ dans l'état « d'anxiété » du rat et de la souris (Cheng et al., 1994 ; Silvestre et al., 1996 ; Kennett et al., 1997; Costall and Naylor, 1997). L'inhibition du récepteur 5-HT₄ entraîne des baisses de l'activité locomotrice chez le rat dans des conditions basales (Fontana et al., 1997), chez la jeune souris âgée de 20 à 27 jours (Semenova and Ticku, 1992) et peut atténuer l'hyperlocomotion induite par la cocaïne (Mc Mahon and Cunningham, 1999).

Dans le striatum, le contrôle sérotoninergique des taux de la DA extracellulaire par l'activation du récepteur 5-HT₄ est à la fois décrit comme excitateur ou inhibiteur

(Bonhomme et al., 1995 ; Steward et al., 1996 ; Deurwaerdere et al., 1997).

Finalement, la stimulation du récepteur 5-HT₄ induit une fermeture des canaux ioniques du potassium (Bockaert et al., 1998), ce qui est susceptible de maintenir l'excitabilité des neurones et d'augmenter la libération des neuromédiateurs. En accord avec cette donnée, la stimulation des récepteurs 5-HT₄ conduit à une élévation des taux de 5-HT extracellulaire dans l'hippocampe.

En résumé, au plan neurobiologique, les récepteurs 5-HT₄ sont connus pour intervenir dans l'apprentissage et la mémoire. Leur contribution possible dans les comportements moteurs et l'état d'anxiété est aujourd'hui décrite comme modérée et s'avère peu étudiée. Une seule étude indique que ce récepteur peut intervenir dans l'effet de la cocaïne sur l'activité locomotrice.

De plus, la demande de brevet internationale publiée sous le numéro WO 97/29739 décrit l'utilisation d'antagonistes du récepteur 5-HT₄ pour la préparation d'un médicament destiné à éviter, alléger, supprimer ou maîtriser les effets gastro-intestinaux causés par un inhibiteur sélectif de la ré-assimilation de la sérotonine. La demande de brevet internationale publiée sous le numéro WO 02/11766 décrit l'utilisation d'antagonistes du récepteur 5-HT₄ dans la prophylaxie ou le traitement de certaines conditions cardio-vasculaires.

Le document Bockaert et al (2003) "New view on the ligand-receptor interaction" European Neuropsychopharmacology, vol 13, no. Supplement 4, septembre 2003, décrit que les récepteur 5HT-4 sont caractérisés par l'existence de 9 variant d'épissage. Ce document décrit également l'identification et le test de différents ligands capables de se lier au récepteur et leurs effets sur la signalisation cellulaire.

La publication de la demande internationale WO 00/64441 décrit l'utilisation de composés ayant une activité agoniste sur le récepteur 5-HT4 comme médicament et l'utilisation desdits composés pour la fabrication d'un médicament pour le traitement thérapeutique de la bronchoconstriction.

La publication de la demande internationale WO 00/77199 décrit une molécule d'isolement d'acide nucléique codant le récepteur 5-HT4 humain et des vecteurs d'expressions incorporant ledit acide nucléique pour la préparation d'une drogue.

De façon surprenante, l'inventeur a montré que l'absence du gène codant le récepteur 5-HT₄ de la sérotonine dès la période embryonnaire a rendu les souris adultes moins sensibles à un stress anorexigène que les animaux sauvages. En d'autres termes, certains stress diminuent la prise alimentaire chez des souris sauvages, mais ils sont moins efficaces chez des souris dépourvues du récepteur 5-HT₄. En l'absence de ce récepteur, les souris consomment donc plus d'aliments que des congénères non génétiquement modifiées.

Puisque le stress contribue à l'apparition de l'anorexie, accompagne la boulimie et accroît la sensibilité aux drogues d'abus, l'inventeur a proposé que les ligands du récepteur 5-HT₄ peuvent atténuer la manifestation de ces pathologies (Anorexie, Boulimie, Addiction aux drogues d'abus associées ou non au stress).

Dans la présente invention, on entend par « récepteur 5-HT₄ » l'un quelconque des sous-types (ou variants d'épissage) du récepteur 5-HT₄ tel que les récepteurs 5-HT_{4A}, 5-HT_{4B}, 5-HT_{4C}, 5-HT_{4D}, 5-HT_{4E}, 5-HT_{4F}, 5-HT_{4G}, 5-HT_{4BH} et 5-HT_{4N}.

La présente invention concerne donc l'utilisation d'un agoniste du récepteur 5-HT₄ choisi dans les 6 classes chimiques que sont les indoles, les benzamides, le benzoate, les aryl-cétones les benzimidazolones et les 1,8-naphtalimides ou d'un sel pharmaceutiquement acceptable de cet agoniste pour la préparation d'un médicament destiné à traiter et/ou à prévenir la boulémie et/ou l'obésité, ou d'un antagoniste choisi dans les 6 classes chimiques que sont les carboxylates d'indole, l'imidazolones, les aryl-cétones, les benzimidazolonés et le s1,8-naphtalimides ou inverse agoniste du récepteur 5-HT₄ ou d'un sel pharmaceutiquement acceptable de ceux-ci pour la préparation d'un médicament destiné à traiter et/ou à prévenir l'anorexie ou l'addiction aux drogues d'abus.

Par « une pathologie liée à une conduite obsessionnelle », on entend les pathologies impliquant des troubles alimentaires et, tout particulièrement, l'anorexie, la boulimie et l'addiction aux drogues d'abus, liées ou non au stress.

Le ligand du récepteur 5-HT₄ utilisé dans le cadre de la présente invention pour la préparation d'un médicament destiné à traiter et/ou à prévenir une pathologie liée à une conduite obsessionnelle et/ou l'obésité n'est pas, de préférence, un ligand du récepteur 5-HT₃ et est un ligand spécifique du récepteur 5-HT₄.

Comme l'inventeur a mis en évidence que le récepteur 5-HT₄ est impliqué dans l'effet anorexigène du stress, il est facilement compréhensible pour l'homme du métier que l'utilisation d'un agoniste de ce récepteur permettra de préparer un médicament destiné à traiter et/ou à prévenir la boulimie, alors qu'un antagoniste ou encore, un agoniste inverse de ce récepteur servira pour préparer un médicament destiné à traiter et/ou à prévenir une pathologie liée à une conduite obsessionnelle choisie dans le groupe constitué par l'anorexie et l'addiction aux drogues d'abus.

Dans le cadre de la présente, on entend par :
- Agoniste: toute molécule capable d'engendrer par sa liaison à ses récepteurs, une réponse biologique semblable à un médiateur endogène.
- Antagoniste : toute molécule capable de bloquer l'action des agonistes.
- Agoniste inverse : toute molécule capable d'inhiber l'activité intrinsèque (ou basale) du récepteur.

Bockaert et al. (1997) ont décrit la structure chimique d'agonistes et d'antagonistes du récepteur 5-HT₄. Les agonistes décrits appartiennent à 6 classes chimiques que sont les indoles, les benzamides, le benzoate, les aryl-cétones, les benzimidazolones et les 1,8-naphtalimides. Les antagonistes décrits appartiennent à 5 classes chimiques que sont les carboxylates d'indole, l'imidazolpyridine, les benzoates, les aryl-cétones, les benzimidazolones et les 1,8-naphtalimides. Ces agonistes et antagonistes sont utilisés dans le cadre de la présente invention. Les agonistes inverses décrits dans Claeysen et al., (2001) et dans Joubert et al. (2002) sont également utilisables dans le cadre de la présente invention.

De plus, les demandes de brevet internationales publiées sous les numéros WO 97/29739 et WO 02/11766 et la demande de brevet internationale publiée sous le numéro WO 02/36113 décrivent, respectivement, des antagonistes et des agonistes du récepteur 5-HT₄ qui eux aussi sont utilisables dans le cadre de la présente invention.

Les agonistes utilisables dans le cadre de la présente invention sont choisis dans le groupe comprenant le metoclopramide, le HTF919 (3-(5-methoxy-1*H*-indol-3-ylmethylene)-*N*-pentylcarbazimidamide hydrogen maleate), le LS650155, le BRL 20627, le BRL 24682, le BRL 24924, le cisapride (Carlsson et al., 1997), le ML 1035 (4-amino-5-chloro-2-[2-(methylsulfinyl)-ethoxy]-N-[2-(diethylamino) ethyl]-benzamide hydrochloride), le mosapride (Carlsson et al., 1997), le R076186, le renzapride, le RS 67506 (1-(4-amino-5-chloro-2-methoxyphenyl)-3-[1-(2-methyl sulphonylamino)ethyl-4-piperidinyl]-1-propanone hydrochloride), le cinitapride, le SB 205149, le SC 49518 (N-[exo-(hexahydro-1H-pyrrolizine-1-yl)methyl]-2-methoxy-4-amino-5-chlorobenzamide HCl), le SC 52491, le SC 53116 (4-amino-5-chloro-*N*-[(hexahydro-1*H*-pyrrolizin-1-yl)methyl]2-methoxybenzamide), le SDZ 216454, le TKS 159 (4-amino-5-chloro-2-methoxy-N-[(2S,4S)-1-ethyl-2-hydroxymethyl-4-pyrrolidinyl] benzamide), le Y 34959, le YM 09151 (N-(1-benzyl-2-methylpyrrolidine-3-yl)-5-chloro-2-methoxy-4-methylaminobenzamide, le YM 47813, le zacopride (4-amino-5-chloro-2-methoxy-*N*-(1-azabicyclo[2.2.2]oct-3-yl)benzamide), le ML 10302 (2-piperidinoethyl 4-amino-5-chloro-2-methoxybenzoate), le RS 57639, le SR 59768 (2-[(3S)-3-hydroxypiperidino]ethyl4-amino-5-chloro-2-methoxybenzoate), le ADR 932, le prucalopride (R093877 ; 4-amino-5-chloro-2,3-dihydro-N-[1-(3-methoxy propyl)-4-piperidinyl]-7-benzofurancarboxamide monohydrochloride), le SK 951, le RS 67333 (1-(4-amino-5-chloro-2-methoxyphenyl)-3-(1-n-burtl-4-piperidinyl)-1-propanone), le RS 17017, le RS 56532, le YM 53389, le BIMU1 (3-ethyl-2,3-dihydro-N-[endo-8-methyl-8-azabicyclo(3.2.1)oct-3-yl]-2-oxo-1H-benzimidazole-1-carboxamide), le BIMU8 (endo-N-8-methyl-8-azabicyclo[3.2.1.]oct-3-yl)-2,3-dehydro-2-oxo-3-(prop-2-yl)-1H-benzimid-azole-1-carboxamide), le DAU 6215 ((3-alpha-tropanyl)1H-benzimidazolone-3-carboxamide chloride), le DAU 6236, la 5-methoxytryptamine, la 2-methylserotonine et la 5-hydroxy-N,N-di-methyltryptamine et la 5-carboxamidotryptamine.

Les antagonistes utilisables dans le cadre de la présente invention sont, avantageusement, choisis dans le groupe comprenant le tropisétron (ICS 205 930; [(3a tropanyl)-1*H*-indole-3-carboxylic acid ester]), le RS 100235 (1-(8-amino-7-chloro-1,4-benzodioxan-5-yl)-3-[[3,4-dimethoxyphenyl)prop-1-yl]piperidin-4-yl]propan-1-one), le RS 39606, le A-85380 (3-(2(S)-Azetidinylmethoxy)pyridine), le GR 113808 (1-[2-[(Methylsulphonyl)amino]ethyl]-4-piperidinyl]methyl 1-methyl-1*H*-indole-3-carboxylate), le GR 125487 (1-[(2-[(Methylsulphonyl)amino]ethyl]-4-piperidinyl]methyl 5-fluoro-2-methoxy-1H-indole-3-carboxylate), le GR 138897 ([1-[2-[(Methylsulphonyl)amino]ethyl]-4-piperidinyl]methyl[(2-(3-methyl-1,2,4-oxadiazol-5-yl)phenyl]carbamate), le SB 203186 (1-piperidinyl)ethyl 1H-indole 3-carboxylate), le SDZ 205-557 2-methoxy-4-amino-5-chlorobenzoic acid 2-(diethylamino) ethyl ester, hydrochloride, le LY 353433 (1,(1-methylethyl)-N-(2-(4-((tricyclo[(2-(3.3.1.1^{3,7}]dec-1-ylcarbonyl)amino)-1-piperidinyl)ethyl)-1H-indazole-3-carboxamide), le LY 297582, le RS 23597 (3- (piperidine-1-yl)propyl-4-amino-5-chloro-2-methoxybenzoate hydrochloride, le SB 204070 (1-butyl-4-piperidyl)methyl 8-amino-7-chloro1,4-benzodioxan-5-carboxylate), le DAU 6285 {(*endo*-6-methoxy-8-methyl-8-azabicyclo [3.2.1] oct 3-yl)-2,3-dihydro-2-oxo-1*H*-benzimidazole-1 carboxylate hydrochloride}, le SC 53606 (1-S,8-S)-N-[(hexahydro-1H-pyrrolizin-1-yl)methyl]-6-chloroimidazo[1,2-a]pyridine-8-carboxamide hydrochloride), le SC 56184, le RS 67532 (1-(4-amino-5-chloro-2-(3, 5-dimethoxy benzyloxyphenyl)-5-(1-piperidinyl)-1-pentanone), le GR 125487 (1-[2(methylsufonyl)amino]ethyl]-4-piperidinyl] methyl-5-fluoro-2-methoxy-1H-indole-3-carboxylate hydrochloride), le SB 207058, le SB 207266 (N-[(1-ⁿbutyl-4-piperidinyl)methyl]-3,4-dihydro-2H-[1,3]oxazino[3,2-a]indole-10-carboxamide), le RS 39604 (1-[4-amino-5-chloro-2-(3,5-dimethoxyphenyl)methyloxy]-3-[1[2-methylsulphonylamino]ethyl]piperridine-4-yl]propan-1-one), le RS 1003002 (N-(2-(4-(3-(8-amino-7-chloro-2,3-dihydro-1,4-benzodioxin-5-yl)-3-oxopropyl)pyperidin-1-y1)ethyl)-methanesulfonamide), le ML 10375 (2-(*cis*-3,5-dimethylpiperidino) ethyl 4-amino-5-chloro-2 methoxybenzoate), le SB 207710 (1-butyl-4-piperidinyl)methyl-8-amino-7-iodo-1,4-benzodioxan-5-carboxylate), le SB 205800 (N-(1-butyl-4-piperidyl)methyl-8-amino-7-chloro-1,4-benzodioxan-5-carboxamide), le N 3389, le FK 1052 et le R 50595.

Les agonistes inverses utilisables dans le cadre de la présente invention sont choisis dans le groupe comprenant RO 116-2617, RO 116-0086 et RO 116-1148.

La quantité de ligand du récepteur 5-HT₄ ou d'un sel pharmaceutiquement acceptable de ce ligand efficace dans le traitement et/ou la prévention d'une pathologie liée à une conduite obsessionnelle et/ou de l'obésité dépendra de la nature de ce désordre et pourra être déterminée par des techniques expérimentales et cliniques standards. De plus, des essais *in vitro* peuvent, de façon optionnelle, être employés pour permettre d'identifier les gammes de doses optimales. Les doses efficaces peuvent être extrapolées à partir de courbes dose-réponse obtenues sur des systèmes de test *in vitro* ou sur modèle animal.

Les compositions pharmaceutiques destinées à traiter et/ou à prévenir une pathologie liée à une conduite obsessionnelle et/ou de l'obésité comprenant un ligand du récepteur 5-HT₄ seront préparées en accord avec les pratiques pharmaceutiques standards. Ces compositions pharmaceutiques se présentent sous une forme appropriée pour une administration par voie parentérale, par voie orale, par voie rectale, par voie nasale, par voie transdermique, par voie pulmonaire, par voie centrale ou par voie systémique.

Ces compositions pharmaceutiques contiennent, outre le ligand du récepteur 5-HT₄ ou le sel pharmaceutiquement acceptable de ce ligand, au moins un véhicule pharmaceutiquement acceptable choisi en fonction de la voie d'administration et de la forme d'administration.

Dans la restitution, dans des aires ciblées du cerveau d'un récepteur 5-HT₄ « sauvage » pour traiter ou prévenir des pathologies liées à des conduites obsessionnelles et/ou de l'obésité et, tout particulièrement, des pathologies liées à des conduites obsessionnelles, dans l'hypothèse où ces pathologies seraient causées par une ou plusieurs mutations dans le gène codant le récepteur 5-HT₄ est également décrit.

Par conséquent, la présente décrit l'utilisation d'un acide nucléique codant pour un récepteur 5-HT₄ fonctionnel ou un récepteur fonctionnellement équivalent pour la préparation d'un médicament destiné à traiter et/ou à prévenir une pathologie liée à une conduite obsessionnelle.

Par « récepteur 5-HT₄ fonctionnel", on entend un récepteur 5-HT₄ sauvage présentant des propriétés pharmacologiques normales.

Par "récepteur fonctionellement équivalent", on entend un récepteur dont la séquence en acides aminés est proche de celle du récepteur 5-HT₄ (au moins identique à 90% et, de préference, au moins identique à 95%) et qui est activée par les mêmes agonistes et avec la même intensité que le récepteur 5-HT₄ sauvage.

La molécule d'acide nucléique codant un récepteur 5-HT₄ fonctionnel ou un récepteur fonctionnellement équivalent est, de façon avantageuse, une molécule codant un récepteur 5-HT₄ de mammifères et, tout particulièrement, une molécule codant un récepteur 5-HT₄ humain. Ces séquences peuvent être obtenues dans Genbank sous les numéros :
- pour l'homme : Y09586 (5-HT_{4A}), Y10437 (5-HT_{4B}) Y12506 (5-HT_{4C}), Y15507 (5-HT_{4D}), AJ011371 (5-HT_{4G}) ;
- pour la souris : Y09587 (5-HT_{4A}), Y09585 (5-HT_{4B}), Y09588 (5-HT_{4E}) AJ011370 (5-HT_{4F}) ;
- pour le rat : U20906 (5-HT_{4A}), 020907 (5-HT_{4B}) et AJ011371 (5-HT_{4E}) ;
- pour le cochon d'Inde : Y13585 (5-HT_{4H}).

De par la dégénérescence du code génétique, d'autres séquences d'acide nucléique qui codent substantiellement les mêmes acides aminés peuvent être utilisées dans le cadre de la présente invention. Ces séquences sont, à titre d'exemple et de façon non exhaustive, des séquences nucléotidiques comprenant tout ou partie de l'acide nucléique codant un récepteur 5-HT₄ modifiées au niveau d'un ou plusieurs codons de façon à produire une mutation silencieuse. Les séquences d'acide nucléique utilisables peuvent être obtenues par différentes méthodes connues dans l'état de la technique, par exemple, via l'ADNc obtenu à partir de l'ARNm du récepteur 5-HT₄ après une réverse transcription.

De façon avantageuse, le noyau accumbens, l'amygdale, l'hippocampe et l'hypothalamus sont les structures cérébrales dans lesquelles il est envisagé de restaurer à la fois l'expression du gène codant le récepteur 5-HT₄ et de supprimer les déficits sur la régulation de la prise alimentaire liés à son absence. Seule une restauration simultanée des déficits moléculaires et cellulaires (taux des monoamines endogènes, activité des neurones sérotoninergiques, par exemple) et comportementaux en conséquence d'un rétablissement de l'expression de ce gène permet de valider un lien causal entre le phénotype moléculaire et comportemental.

Afin d'exprimer un récepteur 5-HT₄ fonctionnel ou un récepteur fonctionnellement équivalent avantageusement dans le noyau accumbens, l'amygdale, l'hippocampe et l'hypothalamus, on peut envisager l'utilisation de vecteurs de transfert, tels que des vecteurs non-viraux ou viraux contenant molécule d'acide nucléique codant un récepteur 5-HT₄ fonctionnel ou un récepteur fonctionnellement équivalent.

Parmi les vecteurs viraux utilisables, nous pouvons citer des virus associés aux adénovirus de type 2, des vecteurs issus du lentivirus avec différents pseudotypes, des vecteurs issus des virus de l'immunodéficience féline et des vecteurs issus du "Foamy virus". Les vecteurs viraux utilisés sont débarrassés de tout effet pathogène et/ou toxique.

L'expression de la molécule d'acide nucléique codant un récepteur 5-HT₄ fonctionnel ou un récepteur fonctionnellement équivalent contenue dans lesdits vecteurs est avantageusement placée sous le contrôle de promoteurs adéquats, en fonction du tissu cible. Pour l'expression d'un récepteur 5-HT₄ fonctionnel ou un récepteur fonctionnellement équivalent dans le noyau accumbens, l'amygdale, l'hippocampe et/ou l'hypothalamus, un promoteur avantageusement utilisé peut être le promoteur du gène codant le transporteur de capture de la dopamine pour le noyau accumbens.

Dans une deuxième forme de mise en oeuvre, la présente invention concerne une méthode pour identifier un composé biologiquement actif susceptible d'être utilisé dans le traitement et/ou la prévention d'une pathologie liée à une conduite obsessionnelle et/ou de l'obésité et, plus particulièrement, un composé biologiquement actif pour le traitement et/ou la prévention d'une pathologie liée à une conduite obsessionnelle caractérisée en ce qu'elle comprend les étapes suivantes :
a) la mise en contact du récepteur 5-HT₄ ou d'un récepteur fonctionnellement équivalent avec ledit composé biologiquement actif, et
b) la détermination si ledit composé biologiquement actif est capable de moduler l'activité basale du récepteur 5-HT₄ ou d'un récepteur fonctionnellement équivalent.

Par "composé biologiquement actif", on entend tout composé chimique naturel ou synthétique capable d'atténuer les symptômes d'une pathologie liée à des conduites obsessionnelles après son administration.

L'étape (a) de la méthode objet de la présente invention peut, tout d'abord, comprendre les étapes suivantes :
i) la mise en culture de cellules qui expriment un récepteur 5-HT₄ fonctionnel ou un récepteur fonctionnellement équivalent, et
ii) l'incubation desdites cellules avec ledit composé biologiquement actif.

Les termes "récepteur 5-HT₄ fonctionnel" et "un récepteur fonctionnellement équivalent" sont tels que définis précédemment.

Avantageusement, les cellules cultivées à l'étape (i) de la méthode objet de la présente invention sont des cellules qui surexpriment un récepteur 5-HT₄ fonctionnel ou un récepteur fonctionnellement équivalent à leur surface. Toute cellule capable de surexprimer un récepteur 5-HT₄ fonctionnel ou un récepteur fonctionnellement équivalent à sa surface est utilisable dans le cadre de la présente invention. A titre d'exemple et de façon non exhaustive, les cellules embryonnaires HEK 293 humaines peuvent être citées.

Une molécule d'acide nucléique codant un récepteur 5-HT₄ fonctionnel ou un récepteur fonctionnellement équivalent ou un vecteur peut être utilisé pour transformer les cellules qui surexpriment un récepteur 5-HT₄ fonctionnel ou un récepteur fonctionnellement équivalent.

Le vecteur qui peut être utilisé dans le cadre de la présente invention pour transformer des cellules surexprimant un récepteur 5-HT₄ fonctionnel ou un récepteur fonctionnellement équivalent comprend au moins une molécule d'acide nucléique codant un récepteur 5-HT₄ fonctionnel ou un récepteur fonctionnellement équivalent telle que décrit ci-dessus, avantageusement associée à des séquences contrôle adaptées au procédé d'expression ou de production desdits récepteurs dans un hôte cellulaire. Le vecteur utilisé est choisi en fonction de l'hôte (cellules cultivées) dans lequel il doit être transféré ; il peut s'agir de tout vecteur tel qu'un plasmide. La préparation de ces vecteurs tout comme la production ou l'expression d'un récepteur dans un hôte cellulaire peuvent être réalisées par toutes les techniques de la biologie moléculaire et de l'ingénierie génétique bien connues de l'homme du métier.

Un composé capable de moduler l'activité de base d'un récepteur est soit un agoniste, soit un agoniste inverse, soit un antagoniste dudit récepteur. La liaison d'un agoniste, d'un agoniste inverse ou d'un antagoniste à un récepteur entraîne des changements dans la conformation de ce récepteur et, au sein de la cellule, une transduction du signal par l'intermédiaire de seconds messagers est observée. Par conséquent, le but de l'étape (b) de la méthode objet de la présente invention consiste à mesurer, par tout moyen adapté, l'affinité entre le récepteur et le composé biologiquement actif, après leur mise en contact.

De façon avantageuse, la modulation de l'activité basale du récepteur 5-HT₄ ou d'un récepteur fonctionnellement équivalent peut être mesurée via l'activation (pour un agoniste) ou l'inhibition (pour un antagoniste ou pour un antagoniste inverse) de la transduction du signal à partir du récepteur 5-HT₄. L'homme du métier connaissant la cascade d'événements induite lors de la transduction du signal à partir de ce récepteur sera capable de déterminer des méthodes et des conditions adéquates pour mesurer l'activation ou l'inhibition de la transduction du signal à partir du récepteur 5-HT₄, par exemple, en mesurant la quantité d'AMPc avant et après la mise en contact avec le composé fonctionnellement actif.

Dans une seconde forme de mise en oeuvre de la méthode objet de la présente invention, les étapes (a) et (b) peuvent être réalisées en fixant un ou plusieurs récepteurs 5-HT₄ ou récepteurs fonctionnellement équivalents sur une ou plusieurs membranes. Les récepteurs 5-HT₄ ou les récepteurs fonctionnellement équivalents peuvent également être intégrés dans un biosenseur. Dans un tel système, il est possible de visualiser en temps réel les interactions entre le composé à tester et le récepteur. Un des partenaires du couple récepteur/ligand est fixé sur une interface, qui peut contenir une matrice couverte de chaînes aliphatiques. Cette matrice hydrophobe peut facilement être recouverte par une couche lipidique par fusion spontanée de liposomes injectés à son contact. Les récepteurs 5-HT₄ ou les récepteurs fonctionnellement équivalents insérés dans les liposomes peuvent alors être integrés dans les biosenseurs. Le composé biologiquement actif est alors analysé par rapport à un ou plusieurs récepteurs 5-HT₄ ou récepteurs fonctionnellement équivalents. La technique du biosenseur permet de plus de mesurer l'affinité de liaison.

une méthode pour traiter et/ou prévenir une pathologie liée à une conduite obsessionnelle consistant à administrer une quantité efficace d'un ligand des récepteurs 5-HT₄ tel que défini précédemment ou d'un acide nucléique codant un récepteur 5-HT₄ fonctionnel ou un récepteur fonctionnellement équivalent tel que défini précédemment est également décrite.

Les résultats obtenus par la Demanderesse ont permis de mettre en évidence que les récepteurs 5-HT₄ contrôlent les taux de leptine et peut, de ce fait, intervenir dans le contrôle de l'obésité. En effet, les présents travaux ont montré que l'absence des récepteurs 5-HT₄ induit une diminution des taux de leptine. Cette implication dans l'obésité est possible car une obésité a été observée chez des souris mutantes privées du récepteur 5-HT₄ âgées de plus de 6 mois et donc plus âgées que celles utilisées dans la partie expérimentale ci-après.

Par conséquent, l'utilisation d'un ligand du récepteur 5-HT₄ tel que défini précédemment et, plus particulièrement, d'un agoniste du récepteur 5-HT₄ tel que défini précédemment pour la préparation d'une composition pharmaceutique destinée au traitement et/ou à la prévention de l'obésité est décrite. Toutes les formes de mise en oeuvre envisagées pour les compositions pharmaceutiques destinées au traitement et/ou à la prévention des conduites obsessionnelles s'appliquent au traitement et/ou à la prévention de l'obésité.

la restitution, dans des aires ciblées du cerveau d'un récepteur 5-HT₄ « sauvage » pour traiter ou prévenir l'obésité, dans l'hypothèse où cette pathologie serait causée par une ou plusieurs mutations dans le gène codant le récepteur 5-HT₄ est décrite.

Par conséquent, dans la présente l'utilisation d'un acide nucléique codant pour un récepteur 5-HT₄ fonctionnel ou un récepteur fonctionnellement équivalent pour la préparation d'un médicament destiné à traiter et/ou à prévenir l'obésité est décrite. Les différentes formes de mises en oeuvre envisagées ci-dessus (récepteur 5-HT₄ de mammifères, récepteur 5-HT₄ humain, vecteur de transfert viral ou non-viral, etc ...) s'appliquent également au traitement et/à la prévention de l'obésité.

Ainsi, une méthode pour traiter et/ou prévenir l'obésité consistant à administrer une quantité efficace d'un ligand et, tout particulièrement, d'un agoniste des récepteurs 5-HT₄ tel que défini précédemment ou d'un acide nucléique codant un récepteur 5-HT₄ fonctionnel ou un récepteur fonctionnellement équivalent tel que défini précédemment est décrite.

La présente invention concerne une méthode pour identifier un composé biologiquement actif susceptible d'être utilisé dans le traitement de l'obésité et, plus particulièrement, un composé biologiquement actif pour le traitement de l'obésité caractérisée en ce qu'elle comprend les étapes suivantes :
a) la mise en contact du récepteur 5-HT₄ ou d'un récepteur fonctionnellement équivalent avec ledit composé biologiquement actif, et
b) la détermination si ledit composé biologiquement actif est capable de moduler l'activité basale du récepteur 5-HT₄ ou d'un récepteur fonctionnellement équivalent.

Les mises en oeuvre décrites dans le cadre de l'identification d'une molécule biologiquement active dans le traitement et/ou la prévention d'une maladie liée à une conduite obsessionnelle s'applique mutatis mutandis à l'identification d'une molécule biologiquement active dans le traitement et/ou la prévention de l'obésité. Compte-tenu des résultats obtenus par la Demanderesse, il est évident pour l'homme du métier qu'une molécule active dans le traitement et/ou la prévention de l'obésité devra se comporter comme un agoniste du récepteur 5-HT₄.

D'autres avantages et caractéristiques de l'invention apparaîtront des exemples qui suivent concernant l'étude comparative de souris invalidées pour le gène codant le récepteur 5-HT₄ de la sérotonine et de souris sauvages, et où il sera fait références aux dessins en annexe dans lesquels :

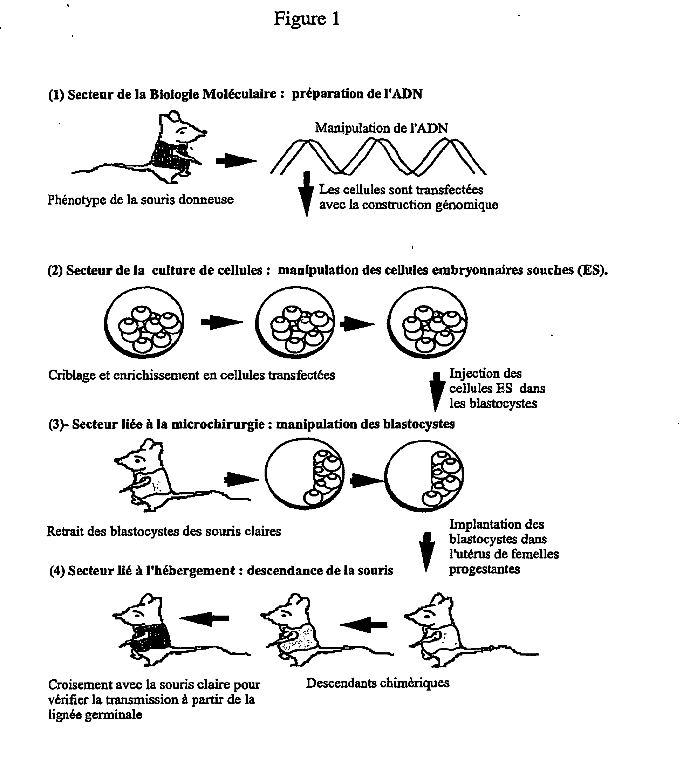

La figure 1 est une représentation schématique des objectifs des secteurs techniques mis en place pour obtenir des souris transgéniques.

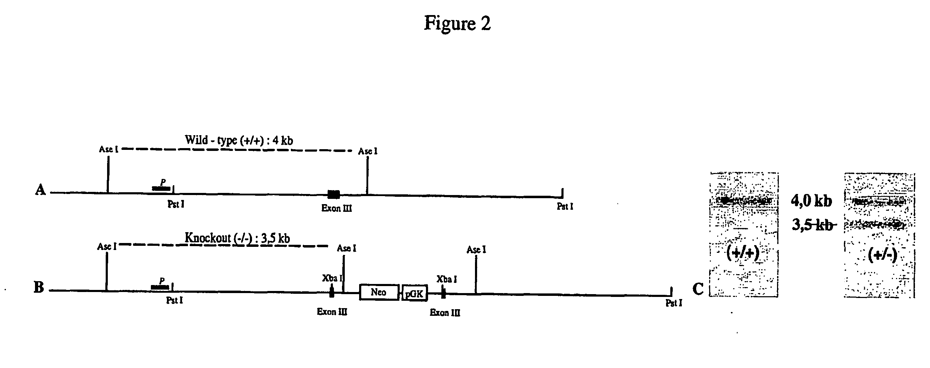

La figure 2 représente l'invalidation du gène codant le récepteur 5-HT₄ de la sérotonine. La figure 2A est une représentation schématique du fragment d'ADN (6,5 kb) codant les domaines transmembranaires II et III du récepteur 5-HT₄ de la sérotonine (exon III). La séquence d'ADN (P) représente la sonde externe pour hybrider un fragment d'ADN Ase I de 4 kb chez les souris sauvages. La figure 2B est une représentation schématique du vecteur de clonage. L'expression du gène codant la néomycine phosphotransférase (Néo) est sous le contrôle d'un promoteur (« phosphoglycerate kinase I promoter : pGK »). Il a été inséré dans un site de restriction enzymatique Xba dans la séquence d'ADN codant pour le domaine transmembranaire III du récepteur 5-HT₄. La taille du fragment d'ADN Ase I qui peut alors être hybridé par la sonde externe est de 3,5 kb et, permet d'identifier l'ADN muté. La figure 2C est un « Southern blot » de l'ADN génomique, des cellules embryonnaires souches, préalablement digéré par l'utilisation de l'enzyme Ase I et hybridé par la sonde externe (P).

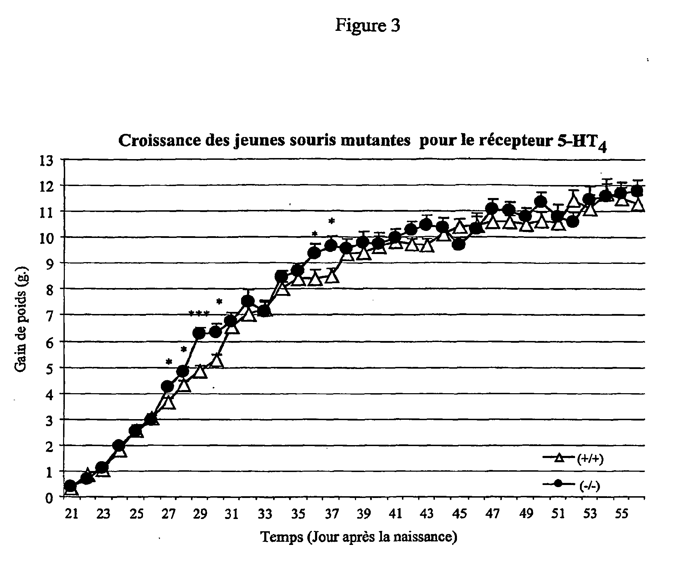

La figure 3 montre que les souris invalidées pour le gène codant le récepteur 5-HT₄ de la sérotonine présentent un gain de poids faiblement augmenté durant une courte période de leur développement. Le gain de poids est la différence entre les poids mesurés un jour donné du développement (21 à 56) et celui du 20^{ième} jour après la naissance. Les données sont les moyennes ± e.s.m. des variations du gain de poids exprimées en g. par jour. Le poids a été mesuré à partir de groupes constitués d'un nombre moyen de 21 sauvages (+/+) et 15 mutants (-/-) pour chaque jour, nés en un an, entre le 20^{ième} jour précédant le sevrage et, le 56^{ième} jour de l'acquisition de leur fertilité après leur naissance. L'analyse statistique a indiqué que les gains de poids des souris mutantes étaient significativement plus élevés que celui des animaux sauvages seulement les jours : 27 (+16%, F_{1,72}=6,54 ; n=40 +/+, n=34 -/-), 28 (+12%, F_{1,67}=4,73 ; n=42 +/+, n=27 -/-), 29 (+30%, F_{1,72}=6,54 ; n=23 +/+, n=18 -/-), 30 (+21%, F_{1,72}=6,54 ; n=20 +/+, n=19 -/-), 36 (+12%, F_{1,40}=4,06 ; n=22 +/+, n=20 -/-) et 37 (+13%, F_{1,40}=6,18 ; n=25 +/+, n=17 -/). Aucune différence significative n'a été détectée entre les deux génotypes pour les autres jours. Les différences significatives entre les génotypes sont marquées par * p<0.05, *** p<0.001.

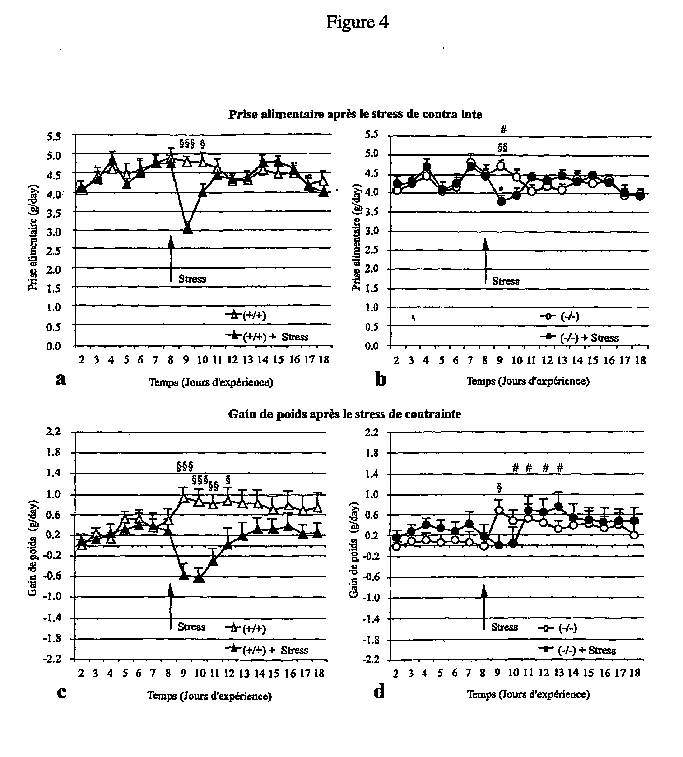

La figure 4 montre que les souris invalidées pour le gène codant le récepteur 5-HT₄ de la sérotonine présentent des réactions de prise alimentaire et de gain de poids anormales après le stress de contrainte ou une immobilisation forcée. Les figures 4a et 4b montrent la prise alimentaire quotidienne chez les souris sauvages (Fig. 4a) et mutantes (Fig. 4b). La prise alimentaire quotidienne est la différence entre la quantité de nourriture évaluée pendant deux jours consécutifs. Les données sont évaluées durant une période d'habituation (8 jours) et de recouvrement (10 jours). Le stress aigu de contrainte de 110 min est appliqué le 8^{ième} jours (flèche). Cette immobilisation forcée a induit une diminution de la prise alimentaire chez les souris sauvages pendant deux jours de la période de recouvrement (Fig. 4a). Cette capacité anorexigène est moins efficace chez les souris dépourvues du récepteur 5-HT₄ (Fig. 4b). Les figures 4c et 4d montrent les variations quotidiennes du gain de poids des souris sauvages (Fig. 4c) et mutantes (Fig. 4d) à partir du premier jour d'isolement (9:00 de la partie éclairée du cycle) de la période d'habituation. Le stress de contrainte a provoqué d'importantes diminutions du gain de poids des souris sauvages (c) mais beaucoup moins ou pas chez les souris mutantes (d). Les données sont les moyennes ± e.s.m. (g./jour) de groupes de souris sauvages non stressées (n=14) ou contraintes (n=18) et d'animaux mutants sans stress (n=17) ou immobilisés (n=16). Les différences significatives entre les animaux stressés ou non sont notées par §§§ p<0,0001 ; §§ p<0,001 ; § p<0,05. Les différences significatives entre les deux génotypes sont marquées par * p<0.05 et les interactions génotype x stress significatives par # p<0,05.

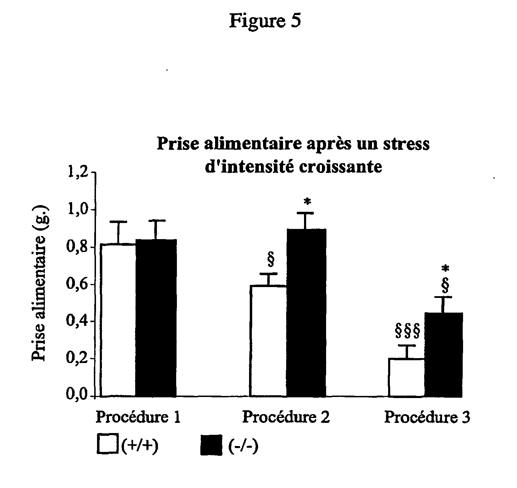

La figure 5 montre que les souris invalidées pour le gène codant le récepteur 5-HT₄ de la sérotonine consomment plus d'aliments que les animaux sauvages après un stress anorexigène d'intensité croissante. En d'autres termes, un paradigme expérimental où la situation est progressivement stressante a induit une diminution de la prise alimentaire chez les souris sauvages alors que l'effet anorexigène a été réduit en l'absence du gène codant le récepteur 5-HT₄. Les données représentent la moyenne ± e.s.m. de la prise alimentaire (g.) dans des contextes expérimentaux nouveaux et/ou aversifs pour les rongeurs (*Procédure 1:* Isolement de 3 h, *Procédure 2:* Labyrinthe en croix surélevées (EPM, 5 min) et isolement de 3 h, *Procédure 3:* Isolement de 96 h, petite incision de la queue, injection de NaCl et, EPM). Les différences significatives entre les souris dépourvues du récepteur 5-HT₄ et, les animaux sauvages sont marquées par * p<0.05. Les différences significatives entre chacune des Procédures sont notées par § p<0,05 ; §§ p<0,01.

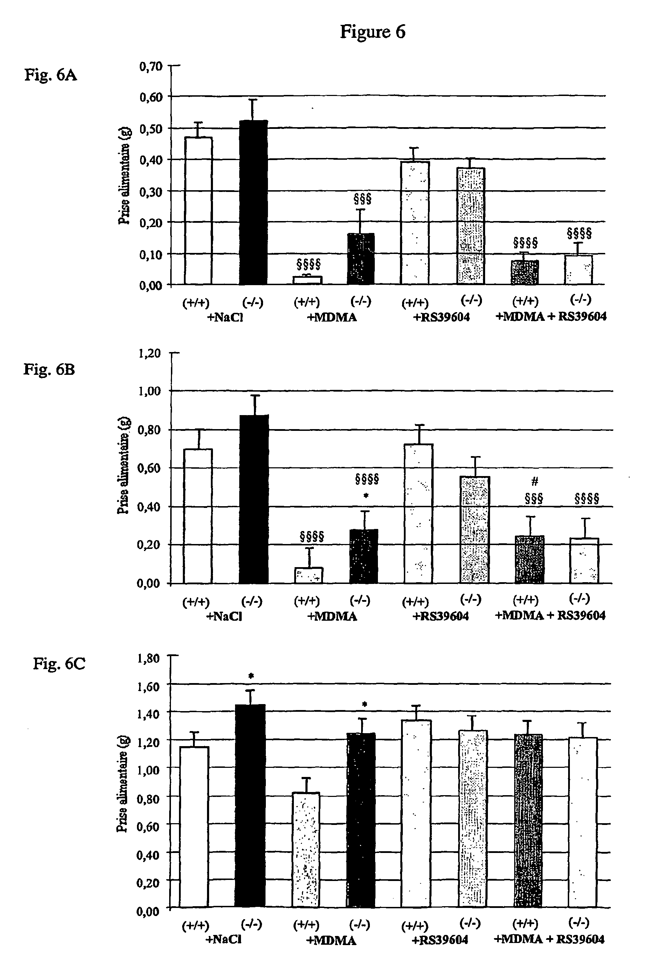

La figure 6 montre que les souris invalidées pour le gène codant le récepteur 5-HT₄ de la sérotonine s'avèrent moins sensibles aux effets anorexigènes du MDMA ou « Ecstasy » 24 après la privation de nourriture. Cette résistance au stress anorexigène de « l'Ecstasy » est reproduite si elle est conjointement administrée avec un antagoniste sélectif du récepteur 5-HT₄ de la sérotonine (RS 39604). En d'autres termes, la forte atténuation de la motivation à consommer des aliments induite par l'injection « d'Ecstasy », en dépit d'une privation de nourriture de 24h, est contrecarrée par l'absence ou l'inhibition des récepteur 5-HT₄. Les données représentent la moyenne ± e.s.m. de la reprise alimentaire (g.) mesurée 30 min (Fig. 6A), 1 h (Fig. 6B) et 3 h (Fig. 6C) après l'injection de NaCl, MDMA (10 mg/kg) et/ou de RS39604 (0,5 mg/kg) chez des souris sauvages (+/+) ou privées du récepteur 5-HT₄ (-/-). *p<0,05 effet significatif du génotype après une analyse de variance suivie du test F de Scheffé. § p<0,05 ; §§§ p<0,001 ; §§§§ p<0,0001 effet significatif du traitement par comparaison avec les animaux de même génotype traités par le NaCl (Anova, Test F de Scheffé). # p<0,05 ; ## p<0,01 effet significatif du traitement par comparaison avec les animaux sauvages traités par le MDMA (Anova, Test F de Scheffé).

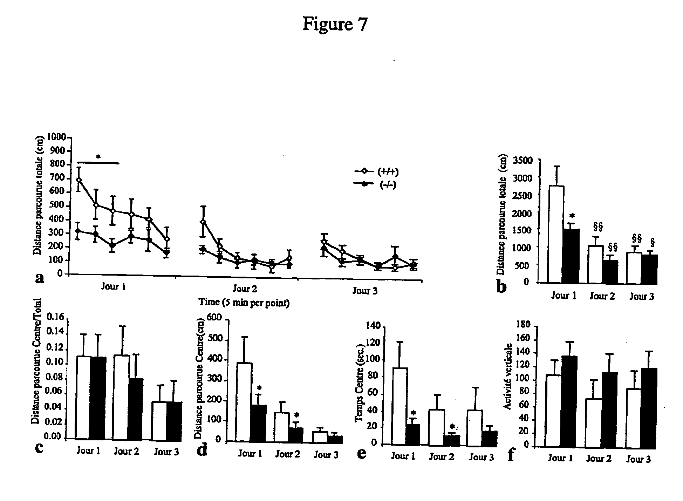

La figure 7 montre que les souris invalidées pour le gène codant le récepteur 5-HT₄ de la sérotonine présentent des déficits de locomotion et/ou d'adaptation à un environnement nouveau, ici « L'open-field ». Les données représentent la moyenne de la distance parcourue (cm) par les souris sauvages (+/+, n=7) et mutantes (-/-, n=7) sur la surface totale (Fig. 7a et 7b) ou dans le centre (Fig. 7c et 7d) de « l'open-field » pendant 30 min et lors de trois jours consécutifs (Jour 1 à 3). Les données de la Fig. 7e représentent le temps moyen passé dans le centre de « l'open-field » lors de trois jours consécutifs. La Fig. 7f représente les données moyennes de l'activité verticale lors de trois jours consécutifs. Les différences significatives entre les génotypes sont marquées par * p<0,05 et, entre les différents jours d'exposition par §§ p<0,01 et § p<0,05.

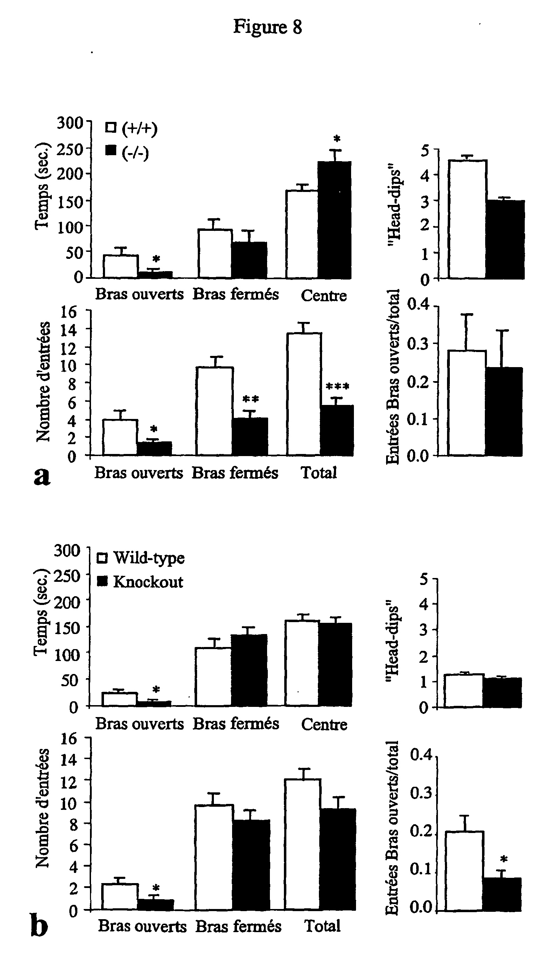

La figure 8 montre que les souris invalidées pour le gène codant le récepteur 5-HT₄ de la sérotonine sont moins réactives au labyrinthe en croix surélevées (EPM) (Fig. 8a) tandis que dans un contexte de stress surajouté, les souris mutantes se sont avérées plus anxieuses (Fig. 8b). Dans l'EPM sans autre stress ajouté, les souris restent moins longtemps dans les bras ouverts (Fig. 8a) et, ont été moins réactives que les animaux contrôles car le nombre d'entrées dans l'ensemble des compartiments de l'EPM sont tous significativement diminués en l'absence du récepteur 5-HT₄ (moyenne ± e.s.m., n=8-9 par génotype, reproduit trois fois dans différents laboratoires). Dans un contexte de stress surajoutés, les souris mutantes ont commencé à réagir au nouvel environnement et montré un comportement plus anxieux que leurs congénères sauvages car elles sont restées et sont entrées moins souvent dans les bras ouverts (moyenne ± e.s.m., n=18-19 par génotype). Les différences significatives entre les génotypes sont marquées par *p<0.05 (Anova).

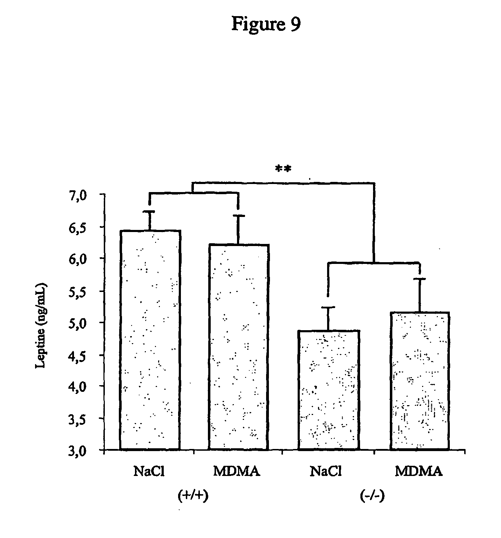

La figure 9 montre que l'absence du gène codant le récepteur 5-HT₄ de la sérotonine a induit une diminution des taux de leptine. Les données représentent la moyenne ± e.s.m. des taux de leptine du plasma exprimées en ng/mL après l'administration de NaCl (0,9 %) ou de MDMA (10 mg/kg) chez des souris (n=8) sauvages (+/+) ou invalidées (-/-) pour le gène codant le récepteur 5HT₄, âgées de 6 à 10 mois en moyenne. **p<0,01 effet significatif du génotype après une analyse de variance (ANOVA) suivie d'un test F de Scheffé.

### I. Matériels et Méthodes.

### I.1. Génération de souris invalidées pour le gène codant le récepteur 5-HT₄ de la sérotonine.

### a. Principe.

La transgenèse est un principe général et consiste en la modification de l'expression d'un gène d'intérêt dans un organisme vivant. Chez la souris, ces modifications nécessitent trois étapes successives (Fig. 1). La première nécessite l'utilisation d'un grand nombre de techniques de la Biologie Moléculaire. Elle consiste en l'obtention d'une construction génomique résultant de la délétion ou de l'insertion du gène d'intérêt, ou encore de la modification par mutagenèse dirigée de quelques bases azotées. La deuxième requiert la transfection de cellules embryonnaires par la transfection de la construction génomique dans des cellules murines embryonnaires totipotentes (« embryonic stem cells » ou ES). L'insertion du gène muté dans leur génome s'effectue par recombinaison homologue au hasard, et de manière stable. Les clones recombinants sont alors sélectionnés par l'utilisation d'un antibiotique (G418 ou néomycine) car un gène de résistance est préalablement inséré lors de la réalisation de la construction génomique. La dernière étape est l'injection des clones positifs dans des blastocystes (Fig. 2).

### b. Vérification de l'absence des récepteurs 5-HT₄ chez les souris mutantes.

Pour vérifier l'absence des récepteurs 5-HT₄ chez les souris mutantes, la technique de radiographie a été utilisée comme précédemment décrite (Compan et al., 1996), à partir de coupes cérébrales des animaux sauvages, hétérozygotes et mutants. Les sites de liaison du récepteur 5-HT₄ ont été marqués par un radioligand sélectif, le [³H]GR113808 (Amersham), antagoniste du récepteur 5-HT₄.

Chez les souris sauvages, une distribution hétérogène des sites de liaison, marqués par le [³H]GR113808 a été observée dans les ganglions de la base, le système limbique ou la formation hippocampale comme déjà observé chez le rat (Waeber et al., 1994, Compan et al., 1996). Aucun marquage spécifique n'a été détecté chez les souris mutantes. La densité des sites de liaison marqués a été diminuée dans l'ensemble des structures cérébrales analysées chez les souris hétérozygotes en comparaison avec les souris sauvages (Tableau 1).

**Tableau 1 : Densité des sites de liaison du récepteur 5-HT₄ marqué par le [³H]GR113808 (0.1 nM) chez des souris adultes de génotype sauvage et hétérozygote.**

| Régions | *B*ₘₐₓ (moyenne ± e.s.m.) en fmol/mg de protéine | |
|---|---|---|
| | Sauvages | Hétérozygotes |
| Tubercules olfactifs | 201 ± 34 | 66 ± 16 (67%) |
| Pallidum Ventral | 171 ± 37 | 53 ± 21 (70%) |
| Noyau Accumbens | 116 ± 28 | 36 ± 16 (68%) |
| Striatum Rostral | 115 ± 15 | 48 ± 11 (48%) |
| Striatum Caudal | 142 ± 25 | 18 ± 10 (85%) |
| Globus Pallidus | 90 t 20 | 14 ± 8 (85%) |
| Hippocampe | 104 ± 11 | 9 ± 8 (91%) |

| | | |
|---|---|---|
| * Considérant une concentration de 1 mg de protéine/10 mg de tissu cérébral. | | |

Le génotype des souris préalablement identifié, a aussi été vérifié par l'utilisation de la technique de polymérisation en réaction par chaîne (« Touch down protocol PCR »). Tous les animaux testés sont nés de couples hétérozygotes et âgés entre 4 et 6 mois. Nous avons obtenu 18 % de souris mutantes sur une période de trois ans. Nous n'avons pas encore d'explication sur la valeur de ce rapport qui ne suit pas les lois de Mendel (Tableau 2).

**Tableau 2 : Rapports mendéliens obtenus après le croisement de souris hétérozygotes.**

| Sauvages | Hétérozygotes | Homozygotes |
|---|---|---|
| 36% (414) | 46% (532) | 18% (209) |

Les souris privées des récepteur 5-HT₄ se développent sans troubles apparents à l'exception d'un gain de poids qui est significativement plus élevé que celui des souris sauvages sur une courte période de leur développement (Fig. 3). Aucune différence significative du poids n'a été détectée chez des souris âgées de 4 à 6 mois (non illustré). Puisque de classiques mécanismes d'adaptation des systèmes neuronaux peuvent survenir au cours du développement, l'hypothèse selon laquelle un contexte inhabituel pourrait induire des troubles de la consommation d'aliments pendant leur période adulte a été formulée par l'inventeur.

### I.2. Tests comportementaux.

La consommation d'aliments et le gain de poids des souris mutantes en comparaison avec des animaux sauvages ont donc été mesurés après l'utilisation du test de contrainte (immobilisation forcée) qui est connu pour constituer un stress anorexigène. De la même manière, la prise alimentaire a été évaluée après l'utilisation de tests de conflits : le labyrinthe en croix surélevées, et l ' « open-field ». Dans ces deux tests expérimentaux, les rongeurs sont confrontés à un conflit entre la motivation à explorer un environnement nouveau et la peur d'espaces ouverts et/ou surélevés.

### a. Animaux.

Les animaux sauvages et mutants sont issus de la lignée Sv 129/Ter (Phillips *et al.,* 1999) et nés de croisements de souris hétérozygotes (+/-) pour la mutation du gène codant le récepteur 5-HT₄ pour conserver la même prédisposition génétique entre les deux génotypes. Les animaux sont élevés et manipulés dans des conditions d'éclairage standard, à la température et aux degrés d'hygrométrie, contrôlés et constants de 22 °C et de 55 % d'humidité relative. Un cycle jour/nuit (12/12 h) a artificiellement été maintenu. La nourriture a la forme de croquettes cylindriques (23 % de protéines ; 3,5 % de matières grasses brutes ; 3 % de cellulose brute ; 7,5 % de cendres brutes ; 12 % d'humidité). Le génotype des souris est identifié par l'utilisation de la technique de la Réaction de Polymérisation en Chaîne ("Polymerase Chain Reaction" ou PCR). Toutes les expériences sont réalisées avec des animaux âgés en moyenne de 4 à 6 mois.

### b. Evaluation de la Prise alimentaire.

*Après le test de contrainte*. Chacune de nos expériences a été divisée en trois phases : une période d'habituation (7 jours), le jour du stress de contrainte (immobilisation pendant 110 min) et une étape de recouvrement (10 jours). Les souris sont divisées en un groupe témoin et contraint. Le jour du stress, des souris sauvages et invalidées pour le gène codant le récepteur 5-HT₄ reçoivent une injection de NaCl 9 %, 1 mg/kg du RS 39604, antagoniste du récepteur 5-HT₄. Parallèlement, un groupe de souris ne reçoit aucun traitement et sont comme les souris précédentes pesées et immobilisée le cas échéant 10 min après le début de leur manipulation. La consommation d'aliments est alors mesurée 2 h 30 min, 3 et 5 h et chaque jour pendant la période de recouvrement.

*Après un isolement de 3h (Procédure 1).* Les animaux sont isolés dans une cage en présence de nourriture et d'eau ad libitum. Le poids de la nourriture et de l'animal est évalué avant et 3 h après l'isolement. La différence entre deux pesées successives révèle la quantité de nourriture ingérée par la souris. Le sol de la cage étant grillagé, il nous a été possible d'évaluer la quantité de nourriture gâchée par chaque animal.

*Après le labyrinthe en croix surélevées (Procédure 2 : stress modéré).* Les mêmes animaux sont regroupés quatre par cage pendant une semaine en salle d'hébergement. Le jour de l'expérimentation, après une période de 30 min, les animaux sont placés dans le labyrinthe en croix surélevées puis isolée afin d'évaluer leur consommation de nourriture 3h après le début du test.

*Après le labyrinthe en croix surélevées anticipé d'une incision (Procédure 3 : fort stress).* Durant une période d'habituation, chaque souris est isolée pendant quatre jours (96 heures) en présence de nourriture et d'eau fournies *ad libitum.* Les poids des souris et des aliments consommés sont quotidiennement mesurés à la même heure pour établir une ligne de base. Le quatrième jour, une petite incision de la queue est effectuée. Un prélèvement de sang est ensuite effectué pendant 10 min pour analyser les taux plasmatiques de corticostérone avant l'injection et le test de conflit. Le labyrinthe en croix surélevées représente aussi un inducteur de stress car il induit une augmentation des taux de corticostérone (Rodgers et al., 1999). L'incision est effectuée dans une pièce différente de la salle du test, toutes les précautions utiles ayant été prises pour éviter tout stress, outre l'incision de la queue et l'immobilisation, avant cette opération. Les échantillons sanguins obtenus sont centrifugés 10 min à 10 000 tours/minute et le plasma est alors stocké à -80°C jusqu'aux dosages ultérieurs. La corticostérone est dosée par l'utilisation de la technique de radioimmunoessai (ICN Clinisciences). Une fois le prélèvement terminé, les animaux sont placés dans la salle d'expérimentation.

Le cinquième jour, les animaux ont reçu une injection intrapéritonéale (i.p.) de NaCl à 9. Les injections sont faites 10 min avant de placer les souris dans le labyrinthe en croix surélevées. Les animaux sont divisés en deux groupes expérimentaux de souris sauvages traitées avec le NaCl (n=8) et, mutantes qui reçoivent aussi une injection de NaCl (n=12). Les souris sont placées dans le centre du labyrinthe en croix surélevées 10 min après l'injection. Une petite incision de la queue est à nouveau pratiquée, 30 min après le test, où les taux de corticostérone atteignent leur maximum et restent élevés pendant quatre heures (Natelson et al., 1987). Suite au test, deux évaluations successives de la prise alimentaire sont effectuées 3 h après le début du test.

*Après* « *l'open-field* ». Nous avons procédé de la même manière que dans le cas de la procédure 2 pour évaluer la prise alimentaire 3 h après le début de la confrontation avec « l'open - field ». En outre, nous avons évalué l'activité motrice des souris un mois après la procédure 2 et 3.

*Après l'administration de drogues.* Chaque drogue, utilisée pour traiter les souris sauvages et invalidées pour le gène codant le récepteur 5-HT₄. a été diluée extemporanément dans une solution saline de NaCl (0,9 %) et injectée de manière systémique (i.p.). Le volume d'injection de chaque traitement est de 200 µL pour 30 g. Les souris sauvages ou invalidées pour le récepteur 5-HT₄ (n = 35) ont reçu les traitements suivants : NaCl, MDMA (ou « Ecstasy » ou 3,4-N-méthylénédioxymethamphétamine, SIGMA, 10 mg/kg), RS 39604, RS 102221/MDMA.

Le MDMA (10 mg/kg, Sigma, licence d'utilisation 9900431 S, V. Compan) et le RS 39604 (0,5 mg/kg), antagoniste du récepteur 5-HT₄, ont été administrés seuls ou en traitement combiné chez des souris sauvages ou privées du récepteur 5-HT₄ (n = 12-17 par génotype et produits pharmacologiques). La dose de 0,5 mg/kg pour le RS 39604 a été sélectionnée car son administration a induit une augmentation de la prise alimentaire chez les souris sauvages nourries *ad libitum* et, reste sans effet chez les animaux mutants pour le récepteurs 5-HT₄ en comparaison des souris traitées par le NaCl ou par différentes doses de RS 39604 (0,01 ; 0,1 ; 1 et 10 mg/kg ; non illustré).

La prise alimentaire des souris sauvages et des souris invalidées pour le gène codant le récepteur 5-HT₄ a été évaluée selon le protocole expérimental décrit par Lucas *et al.,* 1998. Les différentes souris sont isolées durant une période d'habituation de trois jours en présence de nourriture et d'eau fournies *ad libitum* au cours de laquelle les poids des souris et des aliments consommés sont quotidiennement mesurés à la même heure. Le quatrième jour, les souris sont privées de leur nourriture pendant 24 heures. Pour déterminer l'effet des différents traitements sur la prise alimentaire, les drogues sont injectées, comme le NaCl après la période de privation de nourriture. La nourriture est réintroduite dans chaque cage après un intervalle de 10 min pour les autres traitements. Trois pesées successives de la nourriture sont ensuite effectuées 30 min, 1 h et 3 h après la réintroduction de la nourriture.

### c. Tests de conflits.

*Le Labyrinthe en croix surélevées* est constitué de deux aires rectangulaires (L : 57 cm, 1 : 5 cm) qui sont fixées selon un angle droit (90°). L'une des deux aires possède des parois de 15 cm de hauteur et est nommée « bras fermés ». L'autre aire, dépourvue de parois, est appelée « bras ouverts ». Ce dispositif est posé sur un socle à une hauteur de 30 cm au-dessus du sol. Après une période d'habituation de 30 min dans la salle d'expérimentation, chaque animal est placé à l'intersection des deux aires et filmé durant 5 minutes sans que les expérimentateurs soient présents. L'analyse des données consiste en l'évaluation du nombre d'entrées et du temps passé dans les bras ouverts ou fermés par la souris, le temps passé dans le centre et, le nombre de fois où l'animal incline sa tête vers le sol lorsqu'il est présent dans les bras ouverts (« head-dips »). Quand elles sont placées dans le test, les souris sont face à un conflit entre l'exploration d'un nouvel environnement et la peur d'espaces ouverts et en hauteur. Les analyses factorielles mettent en évidence deux types de comportement : l'un lié à l'anxiété et l'autre associé à l'activité motrice (Brunner et al., 1999). Il est communément admis que des animaux anxieux rentrent plus souvent et restent plus longtemps dans les bras fermés et, inversement pour les bras ouverts car les souris sont des animaux nocturnes.

« *L'open-field* » est une aire de 43,2 x 43,2 cm dont les parois sont d'une hauteur de 30 cm. Des capteurs infrarouges sont disposés sur les quatre côtés à une distance de 1,5 cm les uns des autres, ainsi adaptés à la taille des souris. L'enregistrement de la distance parcourue est effectué par un logiciel (MED, Associates Activity Monitor) pendant 30 min à partir du moment où l'animal est placé dans le centre du dispositif expérimental. Ce protocole se répète trois jours de suite. Il est ainsi possible d'évaluer la capacité d'habituation des souris à ce nouvel environnement. Les expérimentateurs ne sont pas présents dans la salle d'expérimentation durant l'enregistrement. Le quadrillage infrarouge permet de faire une analyse fine de plusieurs variables.

### II. Résultats.

### II.1. Hyposensibilité des souris privées du récepteur 5-HT₄ à un stress anorexigène : le stress de contrainte ou immobilisation forcé.

Le stress de contrainte, proposé comme un modèle expérimental d'étude de l'anorexie (Rybkin et al., 1997, Harris et al., 2002) a été utilisé pour tester les limites de résistance des souris invalidées pour le gène codant le récepteur 5-HT₄ à ne pas consommer des aliments après stress. La prise alimentaire et les variations du gain de poids ont été mesurées durant une période d'habituation de 8 jours et de recouvrement de 10 jours (Fig. 4).

Pendant une période d'isolement de 8 jours (période d'habituation), la prise alimentaire des souris dépourvues du récepteur 5-HT₄ n'était pas différente de celle des souris sauvages. Les variations de leur gain de poids pendant la période d'habituation ont été plus faibles que celles des animaux sauvages, comme l'indique l'interaction significative entre le génotype et le temps (F_{1,360}=2,45 ; p<0,05). En d'autres termes, aucune variation significative du gain de poids des souris mutantes n'a été détectée (F_{39,186}=0.94) alors qu'il a été augmenté chez les souris sauvages (F_{29,174}=4,45 ; p<0,001). La prise alimentaire ou les changements de gain de poids entre les deux groupes d'animaux, de même génotype, programmés pour être immobilisés ou non le 8^{ième} jour n'étaient pas significativement différents (Fig. 4 a,b)

Après le stress de contrainte, l'analyse statistique (Anova mesures répétées) a indiqué un effet significatif du stress sur la prise alimentaire au cours du temps (F_{9,567}=13,99 ; p<0,0001) et une interaction génotype x temps significative (F_{9,549}=2.2, p<0.05). L'analyse (« Two-way » Anova) a également révélé que la capacité du stress de contrainte à induire une baisse de la prise alimentaire dépend du génotype les premières 24h qui suivent le stress (F_{1,61}=6,73 ; p<0,05). La consommation d'aliments a significativement été diminuée chez les souris sauvages (-36.4 %, Fig. 4a) et, dans une moindre amplitude, chez les souris mutantes (-19.6 %, Fig. 4b), en comparaison avec les souris de même génotype non-stressées. Une analyse plus détaillée a indiqué que les souris privées du récepteur 5-HT₄ ont significativement consommé plus d'aliments que les souris sauvages 24 h après l'immobilisation (+24 %, p<0,05). Après 48 h, seules les souris sauvages consomment encore moins d'aliments par rapport aux animaux contrôles (-16.4 %, Fig. 4a). Aucun effet significatif du stress sur la prise alimentaire des souris mutantes n'a été détecté 48-h après l'immobilisation forcée (Fig. 4b).

Parallèlement, l'analyse statistique (Anova, mesures répétées) a indiqué un effet significatif du stress sur les variations du gain de poids au cours du temps (F_{9,522}=11,33 ; p<0,0001) et une interaction génotype x temps significative (F_{9,522}=2,38 ; p<0,05). L'analyse statistique (Anova, mesures répétées) a révélé que le stress de contrainte a induit une baisse significative du gain de poids chez les souris sauvages (F_{1,252}=5,76, p<0,05, Fig.4c), mais pas chez les souris invalidées pour le gène codant le récepteur (F_{1,270}=0,014, Fig. 4d). Une analyse statistique détaillée indique que le stress a provoqué des baisses de gain de poids chez les souris sauvages les 4 premiers jours de la période de recouvrement, en comparaison avec les souris contrôles (Fig. 4c). En revanche, le stress de contrainte a eu une moindre efficacité en l'absence du récepteur 5-HT₄ puisque leur gain de poids est resté stable et, plus faible en comparaison avec leurs congénères stressées durant 24-h après l'immobilisation (Fig. 4d).

De plus, les résultats de la Demanderesse indiquent que si le stress de contrainte est précédé d'une injection de RS39604, antagoniste du récepteur 5-HT₄, après l'immobilisation forcée de femelles sauvages, la prise alimentaire des souris sauvages n'est pas modifiée (non illustré). Autrement dit, l'inactivation pharmacologique du récepteur 5-HT₄, a supprimé l'effet anorexigène du stress de contrainte. Parallèlement, l'administration de RS39604 a réduit les pertes de poids des souris sauvages, en comparaison avec les animaux traités par le NaCl (non illustré) (Compan et al., 2003).

### II.2. Hyposensibilité des souris privées du récepteur 5-HT₄ à un stress anorexigène d'intensité croissante.

Les présents résultats indiquent que les souris adultes privées du récepteur 5-HT₄ ont montré moins de sensibilité aux effets anorexigènes d'environnements nouveaux (Fig. 5). L'analyse statistique a montré un effet significatif du génotype (F_{1,47}=5,57 ; p<0,05) et des Procédures utilisées (F_{1,47}=18,55 ; p<0,0001).

Le simple transfert des souris de leur cage habituelle vers les cages d'évaluation de la prise alimentaire n_{'}a pas modifié la consommation d'aliment des souris sauvages et mutantes (*Procédure 1,* Fig. 5). Le *stress modéré (Procédure 2),* une seule exposition dans le labyrinthe en croix surélevées pendant 5 min, connu pour augmenter l'activité de l'axe hypothalamo-hypophysaire, a induit une diminution significative de la prise alimentaire chez les souris sauvages (-33.4 % ; p<0,05 ; n=7). Cela n'a pas été le cas pour les souris mutantes (Fig. 5, n=9). La consommation d'aliments des souris dépourvues du récepteur 5-HT₄ a été significativement plus élevée que celle des animaux sauvages après *le stress modéré* (+48,4 % ; F_{1,15}=7,97 ; p<0,05 ; n=10 ; Fig. 5). En utilisant d'autres séries d'animaux, nos résultats indiquent qu'un *stress intense (Procédure 3)* a entraîné une plus grande diminution de la prise alimentaire chez les souris sauvages en comparaison avec les souris traitées par la *Procédure 2* (-78 %; F_{2,19}=16,11 p<0,0001 ; n=8 ; Fig. 5). Même si la prise alimentaire diminue aussi en l'absence du récepteur 5-HT₄, les souris mutantes consomment plus d'aliments que les animaux sauvages après un *fort stress* (+132 %; F_{1,22}=6.7 ; p=0,0042 ; Fig. 5 ; n=10).

### II.3. L'absence ou l'inactivation du récepteur 5-HT₄ contrecarre l'inhibition de la motivation à consommer des aliments, induite par l'administration de MDMA ou « d'Ecstasy ».

L'analyse statistique globale de la prise alimentaire révèle un effet génotype (F_{1,105}=4,86 ; p<0,05), traitement (F_{1,105}=7,24 ; p<0,001) et temps (F_{3,315}=390,12 ; p<0,0001) significatifs. Les interactions entre le génotype et le traitement (F_{3,105}=2,76 ; p<0,05) et entre les facteurs temps et traitement (F_{9,315}=29,30 ; p<0,0001) sont aussi significatives.

*Dans le cas de la MDMA,* l'analyse statistique montre les effets du génotype (F_{1,59}=11,93 ; p<0,001) et du traitement (F_{1,59}=15,78 ; p<0,001) significatifs qui varient au cours du temps (F_{3,177}=3,97 ; p<0,01) et (F_{3,177}=53,12 ; p<0,0001) respectivement. Nos résultats indiquent que l'administration de MDMA a induit une diminution significative de la prise alimentaire chez les souris sauvages, en comparaison avec les rongeurs de même génotype traitées par le NaCl (Fig. 6). Cet effet est observé 30 min (-95,38 %, Fig. 6A) et 1 h (-88,56 %, Fig. 6B) après son administration et est absent à 3 h (Fig. 6C). Chez les souris dépourvues du récepteur 5-HT₄, comparées aux rongeurs mutants traités par le NaCl, la MDMA induit aussi une diminution significative de la prise alimentaire 30 min (-69,07 %, Fig. 6A) et 1 h (-68,49 %, Fig. 6B) après son administration. Cet effet n'est plus observé 3 h après le traitement des souris (Fig. 6C). De plus, la consommation d'aliments chez les souris dépourvues du récepteur 5-HT₄ est significativement plus élevée 1 h (+645,56 % ; p<0,001, Fig. 6B), et 3 h (+244,23 % ; p<0,05, Fig. 6C) après l'administration de MDMA, en comparaison avec les animaux sauvages ayant reçu le même traitement.

*Pour le RS 39604,* antagoniste du récepteur 5-HT₄, l'analyse statistique ne révèle pas d'effet génotype ₍F_{1,63}=1,37), ni du traitement (F_{1,63}=3,93), ni d'interaction entre les facteurs génotype et traitement significatifs (F_{1,63}=3.93) (Fig. 6).

*Dans le cas de la MDMA combiné au RS 39604, antagoniste sélectif du récepteur 5-HT₄,* l'analyse statistique montre un effet du traitement (F_{1,55}=7,10 ; p<0,05) significatif au cours du temps (F_{3,165}=68,87 ; p<0,0001). Nos résultats indiquent que l'administration combinée de MDMA et du RS 39604 a induit une diminution significative de la prise alimentaire chez les souris sauvages, en comparaison avec les rongeurs de même génotype traités par le NaCl. Cet effet est observé 30 min (-83,72 %, Fig. 6A), et 1 h (-64,89 %, Fig. 6B) après l'administration de MDMA/RS 39604. Aucune variation de la consommation d'aliments n'est observée 3 h après le traitement des animaux (Fig. 6C). Nos résultats sont similaires chez les souris dépourvues du récepteur 5-HT₄. L'administration de MDMA/RS 39604 diminue significativement leur consommation d'aliments 30 min (-82,19 %, Fig. 6A), et 1 h (-72,88 %, Fig. 6B) après l'injection de MDMA/RS 39604 et est sans effet à 3 h (Fig. 6C), en comparaison avec les souris de même génotype. De plus, nos résultats révèlent que la prise alimentaire des souris sauvages est significativement plus élevée 1 h (+206,82 %, Fig. 6B) et 3 h (+51,18 %, Fig. 6C) après l'administration de MDMA/RS 39604, que celle des animaux sauvages ayant reçu la MDMA seule. Cet effet n'est pas révélé chez les souris mutantes pour le récepteur 5-HT₄.

### II.4. Réaction à la nouveauté des souris mutantes pour le récepteur 5-HT₄ dans « l'open-field ».

Les résultats présentés à la Fig. 7 montrent que les souris mutantes parcourent significativement moins de distance dans « l'open-field » que les animaux sauvages seulement le premier jour de leur exposition (F_{1,14}=6,76, p<0.05). Aucune différence significative entre les deux génotypes n'a été détectée le deuxième et troisième jour d'exposition. Les souris privées de récepteurs 5-HT₄ restent aussi significativement moins longtemps dans le centre en comparaison avec leurs congénères sauvages le premier (F_{1,14}=5,50 ; p<0.05) et le deuxième jour (F_{1,14}=4,50 ; p<0.05) d'exposition dans « l'open-field » ce qui suggère un niveau plus élevé d'anxiété chez les souris mutantes (Fig. 7e). Aucune différence significative entre les deux génotypes n'a été détectée pour l'activité verticale (Fig. 7f), ce qui indique que les souris mutantes ne présenteraient pas de déficits d'activité exploratoire.

Ces données montrent une diminution de la réactivité à la nouveauté et/ou de l'activité locomotrice des souris privées du récepteur 5-HT₄ en comparaison avec les souris sauvages (Fig. 7).

### II.5 Etude du niveau d'anxiété des souris privées du récepteur 5-HT₄.

En dépit d'une analogie risquée, l'inventeur a tenu compte de la coexistence des variations de l'anxiété et des troubles alimentaires chez des patients boulimiques. Il a alors analysé si des variations de l'état « d'anxiété » des souris privées du récepteur 5-HT₄ pouvaient être liées à leur persistance à consommer des aliments en dépit de l'application d'un stress anorexigène.

Pour cela, il a utilisé le labyrinthe en croix surélevées, constitué d'aires ou bras fermés et ouverts. Plus les rongeurs passent et entrent dans les bras fermés, plus elles sont considérées comme « anxieuses ». Le principal résultat indique que la procédure 3 (fort stress) a induit une élévation significative du temps passé ou du nombre d'entrée dans les bras fermés, en comparaison avec la procédure 2 (stress modéré) (Fig. 8). Seul le nombre de « head-dips », qui est un index de l'activité exploratoire, diminue significativement après la procédure 3 en comparaison avec la procédure 2 chez les souris sauvages (Fig. 8). Ces résultats suggèrent que les souris dépourvues du récepteur 5-HT₄ présentent un niveau « d'anxiété » plus élevé dans une situation aversive (Procédure 3) par rapport aux souris sauvages.

En outre, la diminution significative du nombre total d'entrée dans les bras ouverts et fermés des souris mutantes dans le cas de la Procédure 2 (Labyrinthe seul) par rapport aux souris sauvages suggère une baisse de leur activité locomotrice et/ou d'un déficit d'adaptation à la nouveauté.

L'étude des comportements alimentaires sous l'influence du stress ne paraît pas pouvoir s'envisager sans considérer l'influence de l'activité locomotrice. Son augmentation peut, de manière évidente, être associée à des besoins énergétiques plus élevés, et ainsi à une consommation d'aliments plus forte et, inversement.

Les données obtenues lors de l'étude de la réaction à la nouveauté (II.4. ci-dessus) associées à celles obtenues par l'utilisation du labyrinthe en croix surélevées suggèrent que la stimulation du récepteur 5-HT₄ est associée à des augmentations de l'activité locomotrice et/ou une augmentation inadaptée de leur réactivité face à des environnements nouveaux. Cette hypothèse renforce celle de l'implication possible du récepteur 5-HT₄ dans les addictions.

### III. Discussion.

Trois types de mécanismes moléculaires sont susceptibles de conférer une résistance aux stress anorexigènes pour les souris privées du récepteur 5-HT₄.

### III.1. Incapacité des systèmes sérotoninergiques à s'adapter au stress afin de suffisamment inhiber la prise alimentaire chez les souris mutantes.

L'inventeur a alors privilégié l'hypothèse d'un déficit de la transmission sérotoninergique après l'application d'un stress en l'absence du récepteur 5-HT₄. L'immobilisation forcée a provoqué une élévation des taux du 5-Hydroxyindol acétique acide (5-HIAA) extracellulaire seulement chez les souris sauvages. Autrement dit, les taux du métabolite principal de la 5-HT restent inchangés en l'absence du récepteur 5-HT₄.

L'hyposensibilité des souris mutantes à un stress anorexigène pourrait ainsi reposer sur l'absence de modification des taux de 5-HIAA extracellulaire (Fig. 9).

### III.2. Augmentation de l'expression des ARNm codant le peptide CART chez les souris mutantes.

L'augmentation de l'expression des ARNm codant le peptide CART (« cocaïne - amphétamine related transcripts », Paradigme expérimental MDMA) observée chez les souris mutantes dans les noyaux accumbens et/ou hypothalamiques en comparaison avec les souris sauvages (non illustré) permettent de formuler l'hypothèse suivante : l'ensemble des ligands du récepteur 5-HT₄ est susceptible de contrôler la consommation d'aliments du fait de leur contrôle de l'expression du peptide CART.

L'administration de ce peptide peut induire des effets anorexigènes et orexigènes suivant son administration respective dans les ventricules latéraux ou les noyaux de l'hypothalamus.

### III.3. Baisses des taux de leptine détectées chez les souris mutantes.

Des baisses des taux de leptine détectées chez les souris invalidées pour le récepteur 5-HT₄ (Fig. 9, Paradigmes expérimentaux MDMA) permettent de proposer que les ligands des récepteurs 5-HT₄ par leur contrôle des taux de leptine puissent réguler les variations de consommation d'aliments et de poids.

L'analyse statistique des variations des taux de leptine n'a pas révélé d'effet significatif du traitement par la MDMA (p=0,95). Ces résultats indiquent qu'une seule administration de MDMA ne modifie pas les taux de leptine chez les souris sauvages ou mutantes (Fig. 9).

En l'absence du gène codant le récepteur 5-HT₄, l'analyse statistique des variations des taux de leptine révèle un effet significatif génotype (F_{1,27}=10,42 ; p<0,01). Ces résultats montrent que les taux de leptine sont significativement moins élevés chez des souris invalidées pour le gène codant le récepteur 5-HT₄, en comparaison avec des animaux sauvages (-21 %) comme l'indique aussi l'utilisation du test F de Scheffé (p<0,01) (Fig. 9).

### III.4. Conclusion.

L'effet anorexigène du stress reposerait sur une cascade d'événements dont le premier maillon est une augmentation de l'activité de l'axe hypothalamo-hypophysaire. Il s'ensuit une élévation de la transmission des monoamines (5-HT et DA) qui inhibe la prise alimentaire. En l'état actuel des connaissances, aucun des récepteurs de la 5-HT n'a été montré comme représentant un élément moléculaire responsable de l'effet anorexigène du stress à l'exception d'une seule étude pharmacologique sur le récepteur 5-HT_{2A/2C} (Grignaschi et al., 1993). Il a été très récemment rapporté que le poids des souris mutantes pour le récepteur 5-HT_{2C} diminue de manière comparable à celui des souris sauvages par suite de l'application répétée du stress de contrainte (Clifton et al., 2003).

Les résultats comportementaux mettent en évidence une hyposensibilité des souris dépourvues du récepteur 5-HT₄ à un stress anorexigène, confirmant le contrôle inhibiteur de la 5-HT sur la consommation d'aliments. Ils étayent l'hypothèse de la contribution de la 5-HT dans l'effet anorexigène d'un stress dont le récepteur 5-HT₄ est l'un des médiateurs. Compte tenu des modifications des paramètres sérotoninergiques dans le NAc et l'hypothalamus en l'absence du récepteur 5-HT₄, il est alors possible que le stress n'augmente pas la libération de 5-HT dans une proportion suffisante pour diminuer la prise alimentaire des souris mutantes, après stress.

Des données sont en faveur d'un contrôle positif de la libération de la 5-HT par suite de l'activation du récepteur 5-HT₄. La stimulation du récepteur 5-HT₄ induit une fermeture des canaux ioniques du potassium (Bockaert et al., 1998), ce qui est susceptible de maintenir l'excitabilité des neurones et d'augmenter la libération des neuromédiateurs. En accord avec cette donnée, la stimulation du récepteur 5-HT₄ conduit à une élévation des taux de 5-HT extracellulaire dans l'hippocampe (Ge & Barnes, 1997). Dans des conditions de base (sans stress), les présentes données indiquent une élévation du rapport 5-HIAA/5-HT dans l'hypothalamus et le NAc, ce qui suggère une altération du métabolisme de la 5-HT en l'absence du récepteur 5-HT₄. En outre, ces résultats suggèrent que la baisse possible des taux de la 5-HT, dans l'hypothalamus, après stress, ne serait pas compensée chez les souris privées du récepteur 5-HT₄ bien que la densité du transporteur membranaire de la 5-HT marqué par le citalopram tritié soit plus forte dans le NRD.

De manière intéressante, la leptine, dont l'invalidation génétique rend des souris obèses, peut augmenter le taux de renouvellement de la 5-HT dans l'hypothalamus (Calapai et al., 1999 ; Hastings et al., 2002) ainsi que, la concentration du transporteur de capture de la 5-HT dans le cortex frontal (Charnay et al., 1999). De plus, l'invalidation du gène codant la leptine induit une diminution des taux d'ARNm codant le transporteur membranaire de la 5-HT dans le NRD (Collin et al., 2000). La localisation des récepteurs de la leptine sur les neurones sérotoninergiques du NRD et du noyau du raphé médian de la souris (Finn et al., 2001) représente un argument supplémentaire en faveur d'une interaction entre la leptine et les neurones sérotoninergiques. Dans le même paradigme expérimental d'utilisation du MDMA, les présents résultats indiquent que les taux de leptine sont diminués chez les souris mutantes. En outre, l'injection de MDMA n'a pas induit de variation de la leptinémie. Ces données suggèrent un contrôle positif des taux de leptine par suite de l'activation du récepteur 5-HT₄ après une mise à jeun de 24h, en accord avec les hausses de leptine induites par l'administration de 5-hydroxytryptophane (Yamada et al., 1999). Toutefois, les taux de leptine ne sont pas modifiés chez les souris mutantes pour le récepteur 5-HT_{1B} (Bouwknecht et al., 2001) ou 5-HT_{2C} (Nonogaki et al., 1998). Les présentes données indiquent que la leptinémie est plus basse chez les souris privées du récepteurs 5-HT4 avant et après le stress de contrainte, en comparaison avec les souris sauvages. Comme une élévation des taux de leptine suit celle de la corticostérone (Guerre-Millo, 1997), les présents résultats suggèrent que les effets d'un stress de privation de nourriture sur la leptinémie sont rendus inefficaces en l'absence du récepteur 5-HT₄ ; il en résulterait une plus forte résistance des souris invalidées pour le récepteur 5-HT₄ aux stress anorexigènes.

Puisque la leptine active l'expression du peptide CART dans le noyau paraventriculaire de l'hypothalamus (Kristensen et al., 1998), les variations possibles du peptide CART dans l'hypothalamus des souris invalidées pour le récepteur 5-HT₄ sont en cours d'analyse, en comparaison avec les souris sauvages. Son implication dans la régulation de la consommation d'aliments est variable suivant le site de son injection. Ainsi, l'administration intracérébroventriculaire du peptide CART résulte en une anorexie (Kristensen et al., 1998) alors qu'une administration intratissulaire dans l'hypothalamus provoque une hyperphagie (Abbott et al., 2001). L'effet orexigène d'une injection du peptide CART dans le noyau paraventriculaire de l'hypothalamus décrit par Abbott et al. (2001) est contredite par les observations de Stanley et al. (2001). Les résultats de l'inventeur indiquent que l'injection du peptide CART dans le « shell » du NAc a réduit l'appétit dans le même paradigme expérimental d'utilisation du MDMA. L'ensemble des résultats suggèrent donc que le MDMA inhibe l'appétit par l'activation du récepteur 5-HT₄ entraînant une régulation positive de l'expression du peptide CART et sa libération. Plusieurs données constituent des arguments en faveur d'un tel mécanisme (1) le récepteur 5-HT₄ est localisé sur des neurones contenant du GABA (Compan et al., 1996), (2) le peptide CART est colocalisé avec le GABA dans les neurones du NAc (Scearce-Levie et al., 1999, Scearce-Levie et al., 2001) ; (3) les résultats présentés ici suggèrent une colocalisation régionale de l'expression des ARNm et des protéines peptide CART/ récepteur 5-HT₄ dans le « shell » du NAc. La colocalisation cellulaire et l'estimation de la concentration du peptide dans le NAc après chacun de ces traitements restant toutefois à établir.

### REFERENCES

Abbott CR. Rossi M. Wren AM. Murphy KG. Kennedy AR. Stanley SA. Zollner AN. Morgan DG. Morgan I. Ghatei MA. Small CJ. Bloom SR. (2001) Endocrinology, 142, 3457-63.
Ackerman S. Nolan LJ. (1998) CNS Drugs., 9, 135-51.
Adell A. Casanovas JM. Artigas F. (1997) Neuropharmacology, 36, 735-741.
Allison DB. Mentore JL. Heo M. Chandler LP. Cappelleri JC. Infante MC. Weiden PJ. (1999) J. Psychiatry, 156, 1686-96.
Amat J. Matus-Amat P. Watkins LR. Maier SF. (1998) Brain Res., 812, 113-120.
Barnes NM. & Sharp T. (1999) Neuropharmacology, 38, 1083-152.
Barsh GS. Farroq IS. O'Rahilly S. (2000) Nature, 404, 644-51.
Bockaert et al. (1997) Handbook of Experimental Pharmacology, Vol. 129. Serotonergic Neurons and 5-HT receptors in the CNS. Eds. H.G. Baumgarten and M. Göthert. Springer-Verlag Berlin Heidelberg. 439-474.
Bockaert J. Ansanay H. Letty S. Marchetti-Gauthier E. Roman F. Rondouin G. Fagni L. Soumireu-Mourat B. Dumuis A. (1998) C R Acad Sci III., 321(2-3), 217-21.
Bockaert J. Claeysen S. Sebben M. Dumuis A. (1998) Ann N Y Acad-Sci., 861, 1-15.
Bockaert J. Claeysen S. Compan V. Dumuis A. (Revue, 2003, sous presse) 5-HT4 receptor in CNS functions : are they potential therapeutical targets ? Drug Target**.**
Bonasera SJ. Tecott LH. (2000) Pharmacol Ther., 88 (2): 133-42.
Bonhomme N. De Deurwaërdere P. Le Moal M. Spampinato U. (1995) Neuropharmacology, 3, 4269-279.
Bouwknecht JA. Van der Gugten J. Hijzen TH. Maes RA. Hen R. Olivier B. (2001) Psychopharmacology, 153(4), 484-90.
Brunner D. Buhot, M-C. Hen R. Hofer M. (1999) Behav Neurosci., 113, 587-601.
Calapai G. Corica F. Corsonello A. Sautebin L. Di Rosa M. Campo GM. Buemi M. Mauro VN. Caputi AP. (1999) J Clin Invest., 104, 975-82.
Carlsson L. Amos GJ. Andersson B. Drews L. Duker G. Wadstedt G. (1997) J Pharmacol Exp Ther., 282, 220-7.
Casper RC. (1998) Depress Anxiety, 8, 96-104.
Chaouloff F. (2000) J. Psychopharmacol., 14, 139-151.
Charnay Y. Cusin I. Vallet PG. Muzzin P. Rohner-Jeanrenaud F. Bouras C. (2000) Neurosci Lett., 283, 89-92.
Claeysen S. Sebben M. Becamel C. Parmentier M-L. Dumuis A. Bockaert J. (2001) EMBO reports, 21, 61-67.
Clifton PG. Lee MD. Somerville EM. Kennett GA. Dourish CT. (2003) Eur J Neurosci., 17, 185-90.
Collin M. Hakansson-Ovesjo M. Misane I. Ogren SO. Meister B. (2000) Brain Res Mol Brain Res., 81, 51-61.
Compan V. Charnay Y. Daszuta A. Hen R. Bockaert J. (2003) Comptes rendus de la Société de Biologie de Paris**.**
Cheng CHK. Costall B. Kelly M. Naylor RJ. (1994) Eur J Pharmacol., 255, 39-49.
Compan V. Daszuta A. Salin P. Sebben M. Bockaert J. Dumuis A. (1996) Eur J Neurosci., 8, 2591-2598.
Compan V. Dusticier N. Nieoullon A. Daszuta A. (1996) Synapse, 24, 87-96.
Costall B. & Naylor RJ. (1997) Br J Pharamcol., 122, 1105-1118.
De Deurwaerdère P. L'Hirondel M. Bonhomme N. Lucas G. Cheramy A. Spampinato U. (1997) J Neurochem., 68, 195-203.
Di Chiara G. (1995) Drug Alcohol Depend, 38, 95-137.
Donohoe TP. (1984) Life Science, 34(3), 203-218.
Dourish CT. Hutson PH. Kennett GA. Curzon G. (1986) Appetite, 7 Suppl, 127-40.
Dumuis A. Bouhelal R. Sebben M. Cory R. Bockaert J. (1988) Mol. Pharmacol., 34, 880-887.
Erecius LF. Dixon KD. Jiang JC. Gietsen DW. (1996) J Nutri., 126, 1722-1731.
Fairburn CG. Doll HA. Welch SL. Hay PJ. Davies BA. O'Connor ME. (1998) Arch Gen Psychiatry, 55, 233-241.
Finn PD. Cunningham MJ. Rickard DG. Clifton DK. Steiner RA. (2001) J Clin Endocrinol Metab., 86, 422-6.
Foltin RW. & Evans SM. (1999) Pharmacol Biochem Behav., 62, 457-64.
Fontana DJ. Daniels SE. Wong EH. Clark RD. Eglen RM. (1997) Neuropharmacology, 36, 689-96.
Garrow J. (1991) Br Med J., 303, 704-706.
Ge J & Barnes NM. (1996), Br J Pharmacol, 117, 1475-80.
Ge J. Barnes NM. Costall B. Naylor RJ. (1997) Pharmacol Biochem Behav., 58, 775-783.
Gerald C. Adham N. Kao HT. Olsen MA. Laz TM. Schetcher LE. Bard JA. Vaysse PJ. Hartig PR. Branchek TA. (1995) EMBO J., 14, 2806-2815.
Godart NT. Flament MF. Lecrubier Y. Jeammet P. (2000) Eur Psychiatry., 15, 38-45.
Grignaschi G. Mantelli B. Samanin R. (1993) Neurosci Lett., 152, 103-6.
Guerre-Millo M. (1997) Biomed Pharmacother., 51, 318-23.
Guy - Grand B. (1995) Obes. Res., 4, 491S-496S.
Harris RB. Mitchell TD. Simpson J. Redmann SM Jr. Youngblood BD. Ryan DH. (2002) Am J Physiol Regul Integr Comp Physiol. 282, R77-88.
Hastings JA. Wiesner G. Lambert G. Morris MJ. Head G. Esler M. (2002) Regul Pept., 103, 67-74.
Heisler LK. Chu HM. Tecott LH. (1998) Ann N Y Acad Sci., 15: 861:74-8.
Hoebel BG. (1997) Appetite, 29, 119-133.
Inoue T. Tsuchiya K. Koyama T. (1994) Pharmacol. Biochem. Behav., 49, 911-920.
Jiang JC. & Gietzen DW. (1994) Pharmacol Biochem Behav., 47, 59-63.
Joseph MH. & Kennett GA. (1983) Brain Res., 270, 251-257.
Joubert L. Claeysen S. Sebben M. Bessis AS. Clark RD. Martin RS. Bockaert J. Dumuis A. (2002) J Biol Chem., 277, (28) 25502-11
Kelley AE. Swanson CJ. (1997) Behav Brain Res., 89, 107-113.
Kennett GA. Bright F. Trail B. Blackburn TP. Sanger GJ. (1997) Neuropharmacology, 36, 707-712.
Konstandi M. Johnson E. Lang MA. Malamas M. Marselos M. (2000) Pharmacol Res., 41, 341-346.
Koob GF. Nestler EJ. (1997) J Neuropsychiatry Clin Neurosci., 9, 482-497.
Kristensen P. et al. (1998) Nature, 393, 72-6.
Kucksmarski RJ. Flegal KM. Campbell SM. Jonhson CL. (1994) J Am Med Assoc., 272, 205-211.
Lilenfeld LR. et al., (1998) Arch Gen Psychiatry, 55, 603-610.
Lowry CA., Rodda JE. Stafford L. Lightman Ingram CD. (2000) J. Neurosci., 20, 7728-7736.
Lucas G. Di Matteo V. De Deurwaerdère P. Porras G. Martin-Ruiz R. Artigas F. Esposito E. Spampinato U. (2001) Eur. J. Neurosci., 13, 889-890.
Lucas J. Yamamoto A. Scearce-Levie K. Saudou F. Hen R. (1998) J Neurosci., 18, 5537-5544.
Mc Mahon LR. & Cunningham KA. (1999) J Pharmacol Exp Ther., 291, 300-307.
Momose K. Inui A. Asakawa A. Ueno N. Nakajima M. Fujimiya M. Kasuga M. (1999) Diabetes Obes Metab., 5, 281-284.
Morley JE. Levine AS. Rowland NE. (1983) Life Science, 32, 2169-82.
Nonogaki K. Strack AM. Dallman MF. Tecott LH. (1998) Nat Med., 4(10), 1152-6.
Pelleymounter MA. Cullen MJ. Baker MB. Hecht R. Winters D. Boone T. Collins F (1995) Science, 28;269(5223), 540-3.
Phillips T.J. Hen R. Crabbe J.C. (1999) Psychopharmacology 147, 5-7.
Price ML. Lucki I. (2001) J. Neurosci., 21, 2833-2841.
Rodgers RJ. Haller J. Holmes A. Halasz J. Walton TJ. Brain PF. (1999) Physiol. Behav, 68, 47-53
Rybkin II. Zhou Y. Volaufova J. Smagin GN. Ryan DH. Harris RB. (1997) Am J Physiol. 273, R1612-22.
Salamone JD. Cousins MS. Snyder BJ. (1997) Neurosci Biobehav Rev., 21, 341-359.
Samanin R. Garattini S. (1996) Therapie, 51, 107-15.
Saudou F. Hen R. (1994) Medical Chemistry Research, 4, 16-84.
Scearce-Levie K. Viswanathan SS. Hen R. (1999) Psychopharmacology, 141, 154-161.
Scearce-Levie K. Coward P. Redfern CH. Conklin BR. (2001) Trends Pharmacol Sci., 22, 414-20.
Semenova TP. Ticku MK. (1992) Brain Res., 588(2), 229-36.
Sillaber I. Montkowski A. Landgraf R. Barden N. Holsboer F. Spanagel R. (1998) Neuroscience, 85, 415-425.
Silvestre JS. Fernandez AG. Palacios JM. (1996) Eur J Pharmacol, 309, 219-222.
Stanley SA. Small CJ. Murphy KG. Rayes E. Abbott CR. Seal LJ. Morgan DG. Sunter D. Dakin CL. Kim MS. Hunter R. Kuhar M. Ghatei MA. Bloom SR. (2001) Brain Res., 893, 186-94.
Steward LJ. Ge J. Stowe LR. Brown C. Bruton RK. Stokes PRA. Barnes NM. (1996) J. Pharm., 117, 55-62.
Stradford TR. Kelley A. (1997) J Neurosci., 17, 4434-4440.
Stradford TR. Kelley A. (1999) J Neurosci., 19, 11040-8.
Stradford TR. Swanwon CJ. Kelley A. (1998) Behav Brain Res., 93, 43-50.
Taber MT. Fibiger HC. (1997) Neuroscience, 76, 1105-112.
Ulmer C. Engels P. Abdel'Al A. Lubbert H. (1996) Naunyn-Schmied Arch. Pharmacol., 354, 210-212.
Vergoni AV. & Bertolini A. (2000) Eur J Pharmacol., 405 (1-3), 25-32.
Vickers SP. Dourish CT. Kennett GA. (2001) Neuropharmacology, 2, 200-9.
Visselman JO. Roig M. (1985) J Clin Psychiatry, 46, 118-24.
Vilaro MT. Cortès R. Gerald C. Branchek TA. Palacios JM. Mengod G. (1996) Mol Brain Res., 43, 356-360.
Waeber C. Sebben M. Nieoullon A. Bockaert J. Dumuis A. (1994) Neuropharmacology, 33, 527-541.
Yamada J. Sugimoto Y. Ujikawa M. (1999) Eur J Pharmacol., 383, 49-51.
Zahorodna A, Tokarski K, Bijak M. (2000) Pol. J Pharmacol., 52(2), 107-9.

## Revendications

1. Utilisation d'un agoniste du récepteur 5-HT₄ choisi dans les 6 classes chimiques que sont les indoles, les benzamides, le benzoate, les aryl-cétones les benzimidazolones et les 1,8-naphtalimides ou d'un sel pharmaceutiquement acceptable de cet agoniste pour la préparation d'un médicament destiné à traiter et/ou à prévenir la boulémie et/ou l'obésité, ou d'un antagoniste choisi dans les 6 classes chimiques que sont les carboxylates d'indole, l'imidazolones, les aryl-cétones, les benzimidazolones et les 1,8-naphtalimides ou inverse agoniste du récepteur 5-HT₄ ou d'un sel pharmaceutiquement acceptable de ceux-ci pour la préparation d'un médicament destiné à traiter et/ou à prévenir l'anorexie ou l'addiction aux drogues d'abus.

2. Utilisation selon la revendication 1, dans laquelle ledit ligand n'est pas un ligand du récepteur 5-HT₃.

3. Utilisation selon l'une quelconque des revendications 1 ou 2, dans laquelle ledit ligand est spécifique du récepteur 5-HT₄.

4. Méthode pour identifier un composé biologiquement actif susceptible d'être utilisé dans le traitement d'une pathologie liée à une conduite obsessionnelle et/ou l'obésité **caractérisée en ce qu'**elle comprend les étapes suivantes :
a) la mise en contact du récepteur 5-HT₄ ou d'un récepteur fonctionnellement équivalent avec ledit composé biologiquement actif, et
b) la détermination si ledit composé biologiquement actif est capable de moduler l'activité basale du récepteur 5-HT₄ ou d'un récepteur fonctionnellement équivalent.

5. Méthode selon la revendication 4 , dans laquelle ladite étape (a) comprend les étapes suivantes :
i) la mise en culture de cellules qui expriment un récepteur 5-HT₄ fonctionnel ou un récepteur fonctionnellement équivalent, et
ii) l'incubation desdites cellules avec ledit composé biologiquement actif.

6. Méthode selon la revendication 5 , dans laquelle les cellules cultivées à l'étape (i) sont des cellules qui surexpriment un récepteur 5-HT₄ fonctionnel ou un récepteur fonctionnellement équivalent à leur surface.

7. Méthode selon l'une quelconque des revendications 5 ou 6, dans laquelle les cellules cultivées à l'étape (i) sont transformées par une molécule d'acide nucléique codant un récepteur 5-HT₄ fonctionnel ou un récepteur fonctionnellement équivalent ou un vecteur comprenant au moins une molécule d'acide nucléique codant un récepteur 5-HT₄ fonctionnel ou un récepteur fonctionnellement équivalent associée à des séquences contrôle adaptées.

8. Méthode selon la revendication 7, dans laquelle la molécule d'acide nucléique codant un récepteur 5-HT₄ fonctionnel ou un récepteur fonctionnellement est, de façon avantageuse, une molécule codant un récepteur 5-HT₄ de mammifères et, tout particulièrement, une molécule codant le récepteur 5-HT₄ humain.

9. Méthode selon l'une quelconque des revendications 4 à 8, dans laquelle la modulation de l'activité basale du récepteur 5-HT₄ ou d'un récepteur fonctionnellement équivalent est mesurée via l'activation (pour un agoniste) ou l'inhibition (pour un antagoniste ou un agoniste inverse) de la transduction du signal.

10. Méthode selon la revendication 4, dans laquelle, à l'étape (a), les récepteurs 5-HT₄ ou les récepteurs fonctionnellement équivalents sont insérés dans des liposomes et intégrés dans des biosenseurs.

## Claims

1. The use of a agonist of the 5-HT₄ receptor selected from 6 chemical classes that are the indoles, the benzamides, benzoate, the aryl cetones, the benzimidazolones and the 1.8-naphthalimides or a pharmaceutically acceptable salt of this agonist for the preparation of a drug for treating and/or preventing bulimia and/or obesity, or an antagonist selected from 6 chemical classes that are the carboxylates of indole, imidazolpydridine, the benzoates, the aryl cetones, the benzimidazolones and the 1,8 naphthalimides or inverse agonist of the 5-HT₄ receptor or a pharmaceutically acceptable salt thereof for the preparation of a drug for treating and/or preventing anorexia or the addiction to drugs of abuse.

2. The use according to Claim 1, **characterized in that** this ligand is not a ligand of the 5-HT₃ receptor.

3. The use of any one of Claims 1 or 2, **characterized in that** this ligand is specific for the 5-HT₄ receptor.

4. A method of identifying a biologically active compound that can be used in the treatment of a pathology associated with an obsessional behavior and/or obesity, **characterized in that** it comprises the following steps:
a) Placing the 5-HT₄ receptor or a functionally equivalent receptor in contact with this biologically active compound, and
b) The determination of whether this biologically active compound is capable of modulating the basal activity of the 5-HT₄ receptor or of a functionally equivalent receptor.

5. The method according to Claim 4, **characterized in that** said step (a) comprises the following steps:
i) The placing of cells in culture that express a functional 5-HT₄ receptor or a functional equivalent receptor, and
ii) The incubation of these cells with this biologically active compound.

6. The method according to Claim 5, wherein the cells cultivated in stage (i) are cells that overexpress a functional 5-HT₄ receptor or a functionally equivalent receptor on their surface.

7. The method according to any one of Claims 5 or 6, wherein the cells cultivated in stage (i) are transformed by a molecule of nucleic acid coding for a functional 5-HT₄ receptor or a functionally equivalent receptor or a vector comprising at least one molecule of nucleic acid coding for a functional 5-HT₄ receptor or a functionally equivalent receptor associated with adapted control sequences.

8. The method according to Claim 7, wherein the molecule of nucleic acid coding for a functional 5-HT₄ receptor or a functionally equivalent receptor is advantageously a molecule coding for a mammalian 5-HT₄ receptor and in particular a molecule coding for the human 5-HT₄ receptor.

9. The method according to any one of Claims 4 to 8, wherein the modulation of the basal activity of the 5-HT₄ receptor or of a functionally equivalent receptor is measured via the activation (for an agonist) or the inhibition (for an antagonist or for an inverse agonist) of the transduction of the signal.

10. The method according to Claim 4, wherein in step (a) the 5-HT₄ receptors or the functionally equivalent receptors are inserted into liposomes and integrated into biosensors.

## Patentansprüche

1. Verwendung eines Agonisten des 5-HT₄ -Rezeptors, der innerhalb der sechs chemischen Klassen bestehend aus Indolen, Benzamiden, Benzoaten, Acrylketonen, Benzimidazolonen und 1,8-Naphthalimiden gewählt wird, oder eines pharmazeutisch akzeptablen Salzes dieses Agonisten zur Herstellung eines Medikaments zur Behandlung und/oder Vorbeugung von Bulimie und/oder Adipositas, oder eines Antagonisten, der innerhalb der sechs chemischen Klassen bestehend aus Indol-Karboxylat, Imidazolonen, Benzoaten, Acrylketonen, Benzimidazolonen und den 1,8-Naphthalimiden gewählt wird, oder eines inversen Agonisten des 5-HT₄-Rezeptors oder eines pharmazeutisch akzeptablen Salzes davon zur Herstellung eines Medikaments zur Behandlung und/oder Vorbeugung von Anorexie oder Drogensucht.

2. Verwendung gemäß Anspruch 1, **dadurch gekennzeichnet, dass** besagter Ligand kein Ligand des 5-HT₃-Rezeptors ist.

3. Verwendung gemäß einem beliebigen der Ansprüche 1 oder 2, **dadurch gekennzeichnet, dass** besagter Ligand für den 5-HT₄ -Rezeptor spezifisch ist.

4. Methode zur Identifizierung einer biologisch aktiven Verbindung, die in der Behandlung und/oder zur Vorbeugung einer mit zwanghaftem Verhalten in Verbindung stehenden Pathologie und/oder Adipositas verwendet werden kann, **dadurch gekennzeichnet, dass** folgende Schritte enthalten sind:
a) In Kontakt bringen des 5-HT₄ -Rezeptors oder eines funktionell äquivalenten Rezeptors mit der besagten aktiven biologischen Verbindung, und
b) Bestimmung, ob die besagte biologisch aktive Verbindung fähig ist, die Basalaktivität des 5-HT₄ - Rezeptors oder eines funktionell äquivalenten Rezeptors zu modulieren.

5. Methode gemäß Anspruch 4, **dadurch gekennzeichnet, dass** besagter Schritt (a) folgende Schritte enthält:
i) das Kultivieren von Zellen, die einen funktionellen 5-HT₄ - Rezeptor oder einen funktionell äquivalenten Rezeptor ausdrücken, und
ii) die Inkubation dieser Zellen mit der besagten biologisch aktiven Verbindung.

6. Methode gemäß Anspruch 5, wobei die in Schritt (i) kultivierten Zellen sind, welche eine Überexpression eines funktionellen 5-HT₄ Rezeptors oder eines funktionell äquivalenten Rezeptors an ihrer Oberfläche aufweisen.

7. Methode gemäß einem beliebigen der Ansprüche 5 oder 6, wobei die in Schritt (i) kultivierten Zellen durch ein Nukleinsäuremolekül verändert werden, das einen funktionellen 5-HT₄-Rezeptor oder einen funktionell äquivalenten Rezeptor oder einen Vektor kodiert, der zumindest ein Nukleinsäuremolekül enthält, das einen funktionellen 5-HT₄-Rezeptor oder einen funktionell äquivalenten Rezeptor kodiert, in Verbindung mit angepassten Kontrollsequenzen.

8. Methode gemäß Anspruch 7, wobei das einen funktionellen 5-HT₄-Rezeptor oder einen funktionell äquivalenten Rezeptor kodierende Nukleinsäuremolekül vorzugsweise ein einen funktionellen 5-HT₄-Rezeptor von Säugetieren und insbesondere den humanen 5-HT₄ -Rezeptor kodierendes Molekül ist.

9. Methode gemäß einem beliebigen der Ansprüche 4 bis 8, **dadurch gekennzeichnet, dass** die Modulation der Basalaktivität des 5-HT₄ -Rezeptors oder eines funkionell äquivalenten Rezeptors mittels der Aktivierung (für einen Agonisten) oder der Inhibierung (für einen Antagonisten oder einen inversen Agonisten) der Signaltransduktion gemessen wird.

10. Methode gemäß Anspruch 4, wobei in Schritt (a) die 5-HT₄-Rezeptoren oder funktionell äquivalente Rezeptoren in Liposome eingebracht und in Biosensoren integriert werden.
